# EUROPEAN PATENT APPLICATION

(11) **EP 2 472 260 A1**
(43) Date of publication of application: **04.07.2012**
(21) Application number: 11195195.0
(22) Date of filing: 22.12.2011
(51) Int. Cl.: G01N 33/543, G01N 27/327

(54) **Photocurrent detection electrode, manufacturing method, and working electrode substrate**

(30) Priority: 28.12.2010 JP 2010293707; 25.11.2011 JP 2011257332
(71) Applicant: Sysmex Corporation, Kobe-shi, Hyogo 651-0073 (JP)
(72) Inventor: Iwanaga, Shigeki, Hyogo 651-0073 (JP); Suzuki, Seigo, Hyogo 651-0073 (JP); Seike, Masayoshi, Hyogo 651-0073 (JP); Hori, Nobuyasu, Hyogo 651-0073 (JP); Kirimura, Hiroya, Hyogo 651-0073 (JP)
(74) Representative: HOFFMANN EITLE

(57) **Abstract**

To provide an electrode and working electrode substrate capable of detecting a sample substance, and a manufacturing method capable of producing the electrode by simply operations, the present invention provides, in a photocurrent detection electrode, an adhesive layer containing linker molecules interposed between an electrode body constituted by a semiconductor for receiving electrons released from a test substance by photoexcitation, and a metal layer constituted by a metal.

## Description

### FIELD OF THE INVENTION

The present invention relates to s photocurrent detection electrode, manufacturing method, and working electrode substrate. More specifically, the present invention relates to a photocurrent detection electrode, manufacturing method and work electrode useful for detection and quantification of a sample substance such as nucleic acid, protein and the like, and for clinical examination and diagnosis of disease using same.

### BACKGROUND

Clinical examination and diagnosis of disease are carried out by detecting disease-related genes and proteins contained in biological samples using methods such as gene detection, immunological detection and the like. Proposed methods for clinical examination and diagnosis include photochemical detection methods used to detect sample substances such as nucleic acids and proteins via an electric current generated by photoexcitation of a photochemically active labeling substance. Clinical examination and diagnosis requires detection of minute amount of sample substance contained in a sample, that is, high sensitivity detection of the sample substance.

For example, U.S. Patent Publication Nos. 2010/0108539 and 2010/0112578 disclose improved sample substance detection sensitivity via a photocurrent detection chip by providing a metal layer between a semiconductor layer and a capture substance in a photocurrent detection chip provided with a photocurrent detection electrode which includes a semiconductor layer and a capture substance for capturing a sample substance.

When using the photocurrent detection chip disclosed in U.S. Patent Publication Nos. 2010/0108539 and 2010/0112578, however, the detection sensitivity and measurement reproducibility are low on rare occasions. As a result of investigating the causes of low reproducibility and detection sensitivity, the present inventors have discovered that the photocurrent detection chip disclosed in U.S. Patent Publication Nos. 2010/0108539 and 2010/0112578 occasionally have peeling of the metal layer from the semiconductor layer, hence causing the reduction of reproducibility and detection sensitivity. For example, peeling of the metal layer may occur when the metal layer is subjected to a blocking process performed to enhance detection sensitivity by inhibiting non-specific adsorption of matter other than the sample substance by the metal layer. When the sample substance is DNA and a nucleic acid probe is used as the capture substance, hybridization and washing under heating are performed so that the capture substance captures the sample substance and to remove the other substances. Since the adhesion force between the semiconductor and the metal is readily weakened by heat, peeling of the metal layer may occur during hybridization. Thus, there is concern that when peeling of the metal layer has occurred in the photocurrent detection chip disclosed in U.S. Patent Publication Nos. 2010/0108539 and 2010/0112578, the capture substance on the metal layer may be lost and detection sensitivity and reproducibility reduced.

### SUMMARY OF THE INVENTION

The scope of the present invention is defined solely by the appended claims, and is not affected to any degree by the statements within this summary.

In view of the foregoing information, an object of the present invention is to provide a photocurrent detection electrode, manufacturing method, and working electrode substrate which provide a high degree of detection sensitivity and reproducibility.

The present inventors completed the present invention after discovering a resolution to the aforesaid problem by adhering the semiconductor layer and the metal layer using a linker molecule.

(1) A first aspect of the present invention is a photocurrent detection electrode used to photoelectrochemically detect a test substance that releases electrons by photoexcitation, comprising:
   a semiconductor body comprising a semiconductor which receives the electrons originating from the test substance irradiated by excitation light;
   an adhesive layer containing linker molecules formed on the electrode body; and
   a metal layer comprising a metal formed on the adhesive layer.
(2) The electrode according to (1), wherein
   a capture substance for capturing the test substance is immobilized on the metal layer.
(3) The electrode according to (2), wherein
   a part of the metal layer, where the capture substance is not immobilized, is blocked by a blocking agent.
(4) The electrode according to any one of (1) to (3), wherein
   the linker molecule comprises at least one selected from groups including; a silane coupling agent;
   a titanium coupling agent; and
   compounds represented by Equation (1):

   R¹-X-R² (1)

   (where R1 represents a silanol group, a phosphoric acid group, carboxyl group or a thiol group, R2 represents an amino group, thiol group, alkyl group having 1 to 4 carbon atoms, hydroxyl, carboxyl, sulfonic acid group, an epoxy group, methacryloyl group; acryloyl group or vinyl group; X represents equation (2):

   (CH₂)ₘ (2)

   (where m represents an integer 1 to 20) or equation (3): 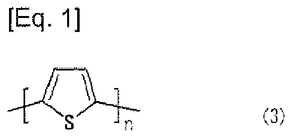 (where n represents an integer 1 to 100), and
   there is an amino bond, ester bond, ether bond, or dithiol bond represented by equation (1)).
(5) The electrode according to any one of (1) to (3), wherein the linker molecule is an amino acid or a compound comprising an amino acid residue.
(6) The electrode according to any one of (1) to (5), wherein
   the metal layer comprises a metal that is dissolved by the electrolyte used in the photoelectrochemical detection of the test substance from which the electrons originate by photoexcitation.
(7) The electrode according to (6), wherein
   the metal layer is at least one metal selected from groups including gold and palladium.

(8) A second aspect of the present invention is a working electrode substrate used to photoelectrochemically detect a test substance that releases electrons by photoexcitation, comprising:
   a substrate body; and
   a photocurrent detection electrode; wherein
   the photocurrent detection electrode comprises:
      a semiconductor body, formed on the substrate body, and comprising a semiconductor which receives the electrons originating from the test substance irradiated by excitation light;
      an adhesive layer containing linker molecules formed on the electrode body; and
      a metal layer comprising a metal formed on the adhesive layer.
(9) The working electrode substrate according to (8), wherein
   a capture substance for capturing the test substance is immobilized on the metal layer.
(10) The working electrode substrate according to (9), wherein
   a part of the metal layer, where the capture substance is not immobilized, is blocked by a blocking agent.

(11) A third aspect of the present invention is a method of manufacturing a photocurrent detection electrode used to photoelectrochemically detect a test substance that releases electrons by photoexcitation, comprising:
   a step of forming an adhesive layer containing linker molecules on an electrode body configured by a semiconductor which accepts the electrons released via photoexcitation, and
   a step of forming as metal layer on the adhesive layer.
(12) The method according to (11), further comprising:
   a step of immobilizing a capture substance for capturing the test substance on the metal layer.
(13) The method according to (12), further comprising:
   a step of blocking a part of the metal layer, where the capture substance is not immobilized, by a blocking agent.

The photocurrent detection electrode and working electrode substrate of the present invention are capable of detecting a sample substance with a high degree of detection sensitivity and reproducibility. The manufacturing method of the present invention is also capable of fabricating the photocurrent detection electrode.

### BRIEF DESCRIPTION OF THE DRAWINGS

FIG. 1 is a perspective view showing a detection device for detecting a sample substance using an embodiment of the working electrode substrate of the present invention.
FIG. 2 is a block diagram showing the structure of the detection device in FIG. 1.
F1G. 3 is a perspective view showing the photocurrent detection chip including the working electrode substrate of the embodiment of the present invention.
FIG. 4A is a cross sectional view on the A-A line of the photocurrent detection chip shown in FIG. 3;
FIG. 4B is a perspective view of the top substrate (corresponding to the working electrode substrate of the embodiment of the present invention) of the photocurrent detection chip shown in FIG. 3 as seen from the bottom side;
FIG. 4C is a perspective view of the bottom substrate of the photocurrent detection chip of FIG. 3 as seen from the top side;
FIG. 5 is a cross sectional schematic view showing a partial example that includes an electrode in a photocurrent detection chip containing the working electrode substrate of the embodiment of the present invention.
FIG. 6 is a cross sectional schematic view showing a partial modification that includes an electrode in a photocurrent detection chip containing the working electrode substrate of the embodiment of the present invention;
FIG. 7A is a plan view of a modification of the top substrate;
FIG. 7B is a plan view of a modification of the bottom substrate (corresponding to the working electrode substrate of the embodiment of the present invention);
FIG. 8A is a plan view of a modification of the top substrate;
FIG. 8B is a plan view of a modification of the bottom substrate (corresponding to the working electrode substrate of the embodiment of the present invention);
FIG. 8C is a perspective view of an interval holding member;
FIG. 9 is a process chart showing the processing sequence of an embodiment of the electrode manufacturing method of the present invention.
FIG. 10 is a process chart showing the processing sequence of an embodiment of the electrode manufacturing method of the present invention.
FIG. 11 is a process chart showing the processing sequence of an embodiment of the sample substance detection method using the electrode of the present invention.
FIG. 12 briefly illustrate the part which includes the working electrode during photocurrent measurement in Experiment 3;
FIG. 13 is a graph showing the results of examining the relationship between the sample substance concentration and photocurrent in Experiment 3.
FIG. 14 is a graph showing the results of measuring the photocurrent in Experiment 5; and
FIG. 15 is a graph showing the results of measuring the photocurrent in Test Example 1.

### DETAILED DESCRIPTION OF THE PREFERRED EMBODIMENTS

The preferred embodiments of the present invention will be described hereinafter with reference to the drawings.

### [Structure of the Detection Device]

An example of a detection device for detecting a sample substance using the working electrode of the embodiment of the present invention is described below with reference to the accompanying drawings.

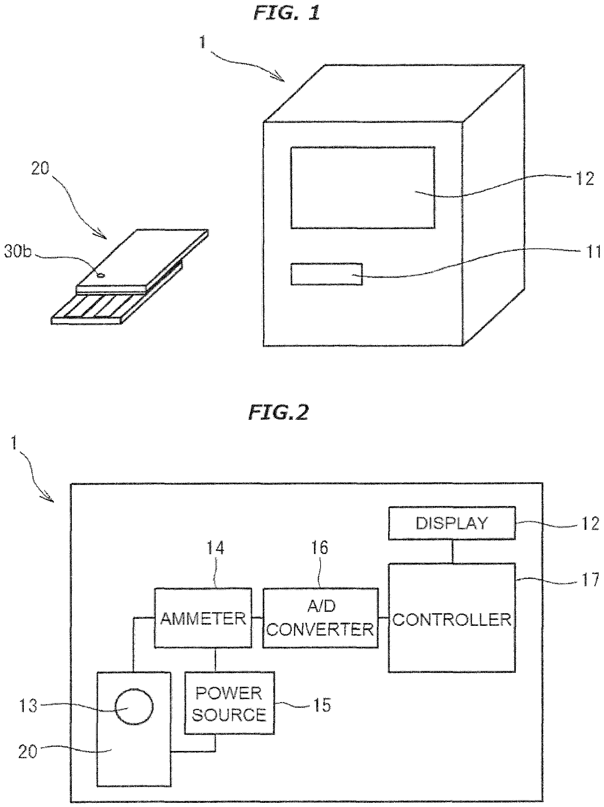

FIG. 1 is a perspective view showing a detection device for detecting a sample substance using an embodiment of the working electrode substrate of the present invention. The detection device 1 uses a photochemically active substance as a labeled substance in a method to photoelectrochemically detect a sample substance.

The detection device 1 is provided with a chip receiver 11 for inserting the photocurrent detection chip 20, and a display 12 for displaying the detection result.

FIG. 2 is a block diagram showing the structure of the detection device in FIG. 1. The detection device 1 is provided with a light source 13, an ammeter 14, a power source 15, an A/D converter 16, a controller 17, and the display 12.
The light source 13 emits light which irradiates the labeled substance present on the working electrode of the photocurrent detection chip 20 to excite the labeled substance. The light source 13 may be a light source which generates excitation light. Fluorescent light, black light, germicidal lamp, incandescent lamp, low pressure mercury lamp, high pressure mercury lamp, xenon lamp, mercury-xenon lamp, halogen lamp, metal halide lamp, LED (white LED, blue LED, green LED, red LED and the like), laser light (carbon dioxide gas laser, dye laser, semiconductor laser), sunlight and the like may be used as the light source. Among these light sources, fluorescent lamp, incandescent lamp, xenon lamp, halogen lamp, metal halide lamp, LED, laser, or sunlight are preferable. The most preferable of these light sources is the laser. The light source also may emit only light of a specific wavelength band via a splitter and bandpass filter as necessary.
The ammeter 14 measures the current flowing within the photocurrent detection chip 20 originating from the electrons released from the excited labeled substance.
The power source 15 supplies a predetermined potential to the electrode provided on the photocurrent detection chip 20.
The A/D converter 16 performs digital conversion of the photocurrent value measured by the ammeter 14.
The controller 17 is configured by a CPU (central processing unit), ROM (read only memory), RAM (random access memory) and the like. The controller 17 controls the operations of the display 12, light source 13, ammeter 14, and power source 15. The controller 17 estimates the amount of labeled substance from the photocurrent value obtained from the digital conversion by the A/D converter 16 based on a previously prepared calibration curve showing the relationship between amount of test substance and the photocurrent, and calculates the amount of sample substance therefrom.
The display 12 then displays information, such as the amount of labeled substance estimated by the controller 17.

### [Structures of the Photocurrent Detection Chip and Working Electrode Substrate]

The structure of the photocurrent detection chip 20, which includes the working electrode substrate of the embodiment of the present invention is described below. Note that, in the present specification, "working electrode substrate" refers to a substrate provided with a working electrode.

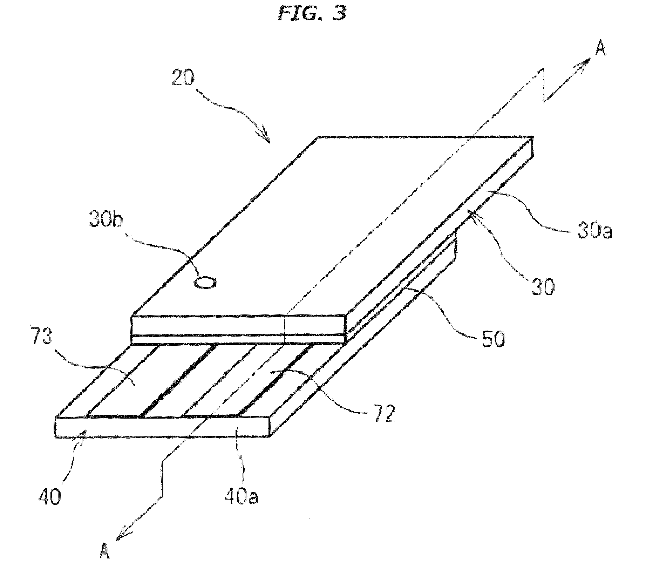

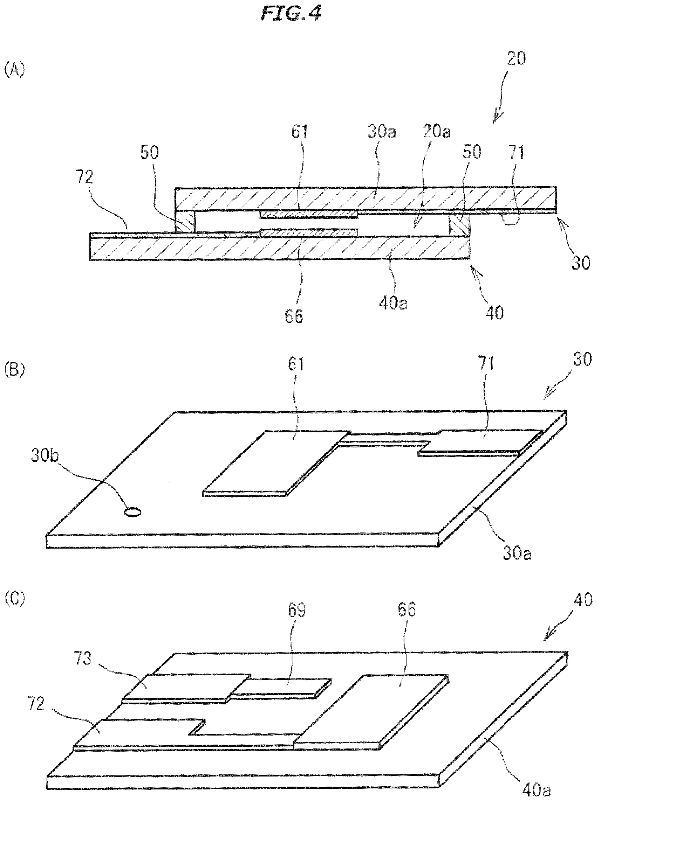

FIG. 3 is a perspective view showing the photocurrent detection chip including the working electrode substrate of the embodiment of the present invention. FIG. 4A is a cross sectional view on the A-A line of the photocurrent detection chip shown in FIG. 3. FIG. 4B is a perspective view of the top substrate (corresponding to the working electrode substrate of the embodiment of the present invention) of the photocurrent detection chip shown in FIG. 3 as seen from the bottom side. FIG. 4C is a perspective view of the bottom substrate of the photocurrent detection chip of FIG. 3 as seen from the top side.

The photocurrent detection chip 20 is provided with a top substrate 30, a bottom substrate 40 which is disposed below the top substrate 30, and an interval holding member 50 which is interposed between the top substrate 30 and the bottom substrate 40. In the photocurrent detection chip 20, the top substrate 30 and the bottom substrate 40 are arranged so as to overlap on one side. The interval holding member 50 is interposed at the overlapping part of the top substrate 30 and the bottom substrate 40.

As shown in FIG. 4B, the top substrate 30 has a substrate main body 30a, and a working electrode 61. The substrate main body 30a has a sample injection inlet 30b for injecting a sample containing the test substance into the interior. The working electrode 61 and an electrode lead 71 connected to the working electrode 61 are formed on the top surface of the substrate main body 30a. In the top substrate 30, the working electrode 61 is disposed on part of one side of the substrate main body 30a (left side in FIG. 4B). The electrode lead 71 extends from the working electrode 61 toward the other side (right side in FIG. 4B) of the substrate main body 30a. The sample injection inlet 30b is on the inner side of the substrate main body 30a from the part where the interval holding member 50 is interposed.

The substrate main body 30a is formed in a rectangular shape. Note that the shape of the substrate main body 30a is not specifically limited and also may be polygonal-shaped, disk-shaped or the like. From the perspective of ease of fabrication and handling of the substrate, the substrate main body 30a is preferably a rectangular shape.

The material constituting the substrate body 30a is not specifically limited, and may be, for example, glass, plastics such as polyethylene terephthalate, polyimide resins, and inorganic materials such as metals. Among these materials, glass is preferable from the perspectives of optical transparency, adequate heat resistance, ensuring smoothness, and low material cost. From the perspective of ensuring sufficient durability, the thickness of the substrate main body 30a is preferably 0.01 to 1 mm, more preferably 0.1 to 0.7 mm, and most preferably about 0.5 mm. In addition, the size of the board body 30a is not specifically limited, but is usually about 20 x 20 mm depending on the number of items to be detected when detecting a wide variety of test substances and sample substances (many items).

The bottom substrate 40 has a substrate main body 40a, working electrode 66, counter electrode 66, and reference electrode 69 as shown in FIG. 4C. The substrate main body 40a has substantially the same rectangular shape and dimensions as the substrate main body 30a of the top substrate 30. The substrate body 40a and the substrate body 30a do not necessarily have the same dimensions.

The material constituting the substrate body 40a may be a material which is permeable to light. This material is not specifically limited, and may be, for example, glass, plastics such as polyethylene terephthalate, polyimide resins, and inorganic materials such as metals. Among these materials, glass is preferable from the perspectives of adequate optical transparency, heat resistance, durability, smoothness, and low material cost. The material, size and thickness of the substrate body 40a is identical to the material, size and thickness of the substrate body 30a of the top substrate 30.

The surface of the substrate main body 40a has a counter electrode 66, an electrode lead 72 connected to the counter electrode 66, a reference electrode 69, and an electrode lead 73 connected to the reference electrode 69. In the bottom substrate 40, the counter electrode 66 is disposed on part of one side of the substrate body 40a (right side in FIG. 4C). The reference electrode 66 is disposed on the substrate main body 40a at a position opposite the counter electrode 66. The electrode lead 72 of the counter electrode 66 and the electrode lead 73 of the reference electrode 69 respectively extend from a part on one side (right side in FIG. 4C) of the substrate main body 40a toward the other side (left side in FIG. 4C). The electrode leads 72 and 73 of the counter electrode 66 and the reference electrode 69 are arranged so as to be mutually parallel at the other side of the substrate main body 40a (left side in FIG. 4C). The electrode leads 72 and 73 extend from the overlapping part of the top substrate 30 and the bottom substrate 40 so as to be exposed to the outside (refer to FIGS. 3 and 4A).

The working electrode 61, counter electrode 66, and reference electrode 69 are described in detail below.

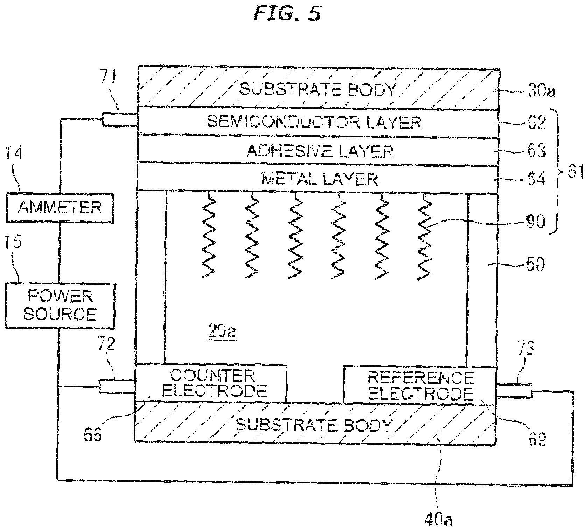

FIG. 5 is a cross sectional schematic view showing a partial example that includes an electrode in a photocurrent detection chip containing the working electrode substrate of the embodiment of the present invention. The working electrode 61 is approximately square in shape. As shown in FIG. 5, the working electrode 61 is configured by a semiconductor layer 62 provided as a working electrode body formed on the substrate main body 30a, an adhesive layer 63 formed on the semiconductor layer 62, a metal layer 64 formed on the adhesive layer 63, and a capture substance 90 immobilized on the metal layer 64. The electrode lead 71 of the working electrode 61 is connected to the semiconductor layer 62.

In the present specification, the concept of the "working electrode," includes an electrode configured by the semiconductor layer 62 acting as the working electrode body, adhesive layer 63, and metal layer 64.

The semiconductor layer 62 is configured by a semiconductor which receives the electrons originating from the test substance irradiated by excitation light. The semiconductor layer 62 functions as a conductive layer and an electron acceptor layer. The semiconductor may be a substance that obtains an energy level by injection of electrons originating from a test substance by photoexcitation. In this case, the "energy level by injection of electrons originating from a test substance by photoexcitation" means a conduction band. That is, the semiconductor may have a lower energy level than the lowest unoccupied molecular orbital (LUMO) of the labeled substance (described later). The semiconductor is not specifically limited, and examples of useful materials include individual semiconductors such as silicon, germanium and the like; oxide semiconductors containing oxides of titanium, tin, zinc, iron, tungsten, zirconium, hafnium, strontium, indium, cerium, yttrium, lanthanum, vanadium, niobium tantalum and the like; perovskite-type semiconductors such as strontium titanate, calcium titanate, sodium titanate, vanadium titanate, potassium niobate and the like; sulfide semiconductors containing sulfides of cadmium, zinc, lead, silver, antimony, bismuth and the like; semiconductors containing nitrides of gallium, titanium and the like; semiconductors formed of selenide of cadmium, lead (for example, cadmium selenide and the like); semiconductors containing cadmium telluride; semiconductors containing phosphide of zinc, gallium, indium, cadmium and the like; semiconductors containing compounds of gallium arsenide, copper - indium - selenide, copper - indium - sulfide and the like; and organic semiconductors or compound semiconductors containing carbon. Note that these semiconductors also may be either true semiconductors or impure semiconductors. Among these examples, oxide semiconductors are preferable. Among true oxide semiconductors, titanium oxide, zinc oxide, tin oxide, niobium oxide, indium oxide, tungsten oxide, tantalum oxide, and strontium titanate are preferable. Among the impure oxide semiconductors, tin-doped indium oxide, and fluorine-doped tin oxide are preferable. The thickness of the semiconductor layer 62 is normally 0.1 to 1 µm, preferably 0.1 to 200 nm, and more preferably 0.1 to 10 nm.

The adhesive layer 63 contains linker molecules. The linker molecules do not inhibit the transfer of electrons between the semiconductor layer 62 and is a compound that does not cause a background current via photoexcitation when used in the detection of the photocurrent, and also may be any compound that bonds to both the semiconductor layer 62 and metal layer 64. That is, the linker molecules may be a compound that mitigates differences of polarity (hydrophilic, hydrophobic) of the semiconductor layer 62 and the metal layer 64. For example, if the semiconductor layer 62 has a higher degree of hydrophilicity compared to the metal layer 64, the linker molecule may have a higher degree of hydrophilicity than the metal layer 64 and a higher degree of hydrophobicity than the semiconductor layer 62. In another example, the linker molecule may be a compound that has a functional group which bonds with the semiconductor of the semiconductor layer 62, and a functional group that bonds with the metal of the metal layer 64. Examples of useful linker molecules include silane coupling agent, titanium coupling agent, amino acid, polyamino acid, peptides, proteins, compounds comprising an amino acid residue, and compounds represented by equation (1):

R¹-X-R² (1)

(where R¹ represents a silanol group, a phosphoric acid group, carboxyl group or a thiol group, R² represents an amino group, thiol group, alkyl group having 1 to 4 carbon atoms, hydroxyl, carboxyl, sulfonic acid group, an epoxy group, methacryloyl group; acryloyl group or vinyl group; X represents equation (2):

CH₂)ₘ (2)

(where m represents an integer 1 to 20) or equation (3):

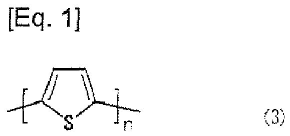

(where n represents an integer 1 to 100), and
may have a compound represented by [having an amide bond, ester bond or dithiol bond] in the molecule represented in equation (1).
Since the silane coupling agent and titanium coupling agent have a functional group for bonding to the semiconductor constituting the semiconductor layer 62, and a functional group for bonding to the metal constituting the metal layer 64, the semiconductor layer 62 and the metal layer 64 can be adhered together by bonding both the semiconductor layer 62 and the metal layer 64, or mitigating the difference in the polarity of the semiconductor layer 62 and the metal layer 64 (hydrophilic, hydrophobic).
In the compound represented by equation (1), R1 and R2 fulfill the role of bonding the semiconductor constituting the semiconductor layer 62 and the metal constituting the metal layer 64, or have properties fulfilling the role of mitigating the difference in polarity of the semiconductor layer 62 and the metal layer 64 (hydrophilicity, hydrophobicity). R1 and R2 may have a substituent insofar as such substituent doe not interfere with the objects of the present invention. Usable alkyl groups having 1 to 4 carbon atoms may include, for example, a methyl group, ethyl group, n-propyl group, isopropyl group, n-butyl group, tert-butyl group and the like. Among these, a methyl group is preferable from the viewpoint of rigidly adhering the metal layer 64 and the semiconductor layer 62.
Amino acids and amino acid residues have carboxyl group and amino group (or imino group) at terminals to provide excellent reactivity for both the semiconductor layer 62 and the metal layer 64. Therefore, when a compound containing an amino acid or amino residue group is used as a linker molecule, the metal layer 64 and the semiconductor layer 62 can be stronger adhered via an adhesive layer 63 containing such a linker molecule.
The amino acid may be an, acidic amino acid, neutral amino acid, and basic amino acid. The amino acid may be an L body amino acid or R body amino acid insofar as the amino acid does not interfere with the object of the present invention. Amino acids include, but are not limited to, for example, cysteine, lysine, alanine, arginine, asparagine, aspartic acid, glutamine, glutamic acid, glycine, histidine, isoleucine, leucine, methionine, phenylalanine, proline, serine, threonine, tryptophan, tyrosine, valine and derivatives thereof and the like. These amino acids, insofar as they do not interfere with the purpose of the present invention, may have substituents, for example, hydroxyl group, formyl group, pyrrolinyl group, phosphate group, selenol group and the like. Among such amino acids, cysteine and lysine are preferable, and lysine is most preferable from the perspectives of strong adherence between the metal layer 64 and the semiconductor layer 62, and effectively suppressing the generation of noise.
Compounds containing amino acid residues include, but are not limited to, for example, polyamino acids; and compounds obtained by polymerizing a monomer of amino acid and non-amino acid. Amino acid residues, insofar as they do not interfere with the object of the present invention, may be amino acid residues derived from the L-group amino acids, and amino acid residues derived from R-group amino acids. Compounds containing amino acid residue, may have both the amino acid residue from an L-group amino acid and the amino acid residue from an R-group amino acid, or only one other amino acid residue from an L-group amino acid or an amino acid from an R-group amino acid. The amino acid residues are not specifically limited, and may be, for example, amino acid residue derived from the previously mentioned amino acids. Among such amino acid residues, cysteine residue and lysine residue are preferable, and lysine residue is most preferable from the perspectives of strong adherence between the metal layer 64 and the semiconductor layer 62, and effectively suppressing the generation of noise.
Polyamino acid is a compound polymerized via a peptide bond using amino acids as monomers. Polyamino acids may be high molecular weight compounds (homopolymer) obtained by polymerization of one type of monomer (amino acid), and may be compounds (copolymers) obtained by polymerization of two or more types of monomers (amino acids). Homopolymers are not specifically limited, and may be, for example, polylysine and the like. Copolymers are not specifically limited, and may be, for example, peptides, proteins and the like. Note that in cases having an amino group or carboxyl group of amino acids in a non-α position as a monomer constituting the polyamino acid, insofar as the poly amino acid does not interfere with the purpose of the present invention, the polyamino acid may have a peptide bond between an α position amino group (or imino group) and a non-α-position carboxyl group, or a peptide bond between an α-position carboxyl group and a non-α position amino acid (or imino group).
These linker molecules may be used individually or as mixtures of two or more types. Since the working electrode 61 is provided with such an adhesive layer 63 in the present embodiment, there is stronger adhesion between the metal layer 64 (described later) and the semiconductor layer 62, acting as the working electrode body, intermediated by the adhesive layer 63. As a result, the metal layer 64 is unlikely to peel away when the capture substance captures the test substance during detection of the test substance, and when a blocking process is performed to improve detection sensitivity. Therefore, insofar as the photocurrent detection chip 20 includes the working electrode substrate of the present embodiment, sample substance detection with high reproducibility is obtained. Note that titanium and chromium are conventionally used as means for adhering the semiconductor and the metal. The present inventors have discovered that severe noise is generated in electrodes used in photocurrent detection when titanium and chromium are used to adhere the semiconductor layer and metal layer. However, noise generation is suppressed when an adhesive layer containing linker molecules is used as in the present embodiment. The working electrode substrate of the present embodiment is therefore advantageous from the perspective of ensuring sufficient detection sensitivity.

The metal layer 64 is configured by a metal capable of immobilizing the capture substance 90. The metal is preferably a metal capable of covalent bonding with the capture substance 90. The metal is also preferably a metal that is dissolved by the electrolyte used in the photoelectrochemical detection of the test substance which the electrons originate by photoexcitation. Examples of useful metals include gold, platinum, silver, palladium, nickel, mercury, rhodium, ruthenium, copper or alloys thereof. Among these, gold and palladium are preferred. From the perspective of ensuring sufficient thickness to immobilize the capture substance, the thickness of the metal layer 64 is 1 nm or greater, and preferably 2 nm or greater, but less than 20 nm from the perspective of suppressing the generation of a background current originating from the metal. When the thickness of the metal layer 64 is less than 2 nm, the metal layer 64 is formed in the shape of an island on the adhesive layer 63. Even in this case, however, it is possible to ensure a metal layer 64 which has sufficient surface area to immobilize the capture substance. The blocking layer (described later), on the other hand, is formed by the reaction between the blocking agent and the metal of the metal layer 64. Accordingly, when a blocking layer is provided on the metal layer 64 to improve detection sensitivity, it is preferable that the entire surface of the adhesive layer 63 covers the metal layer 64 from the perspective of obtaining sufficient improvement of detection sensitivity. However, this does not apply when an adhesive layer 63 is used which is capable of immobilizing the blocking agent.

A capture substance 90 is immobilized on the surface of the metal layer 64 (refer to FIG. 5). The capture substance 90 is a substance for capturing a test substance. The test substance can be brought to the vicinity of the working electrode 61 via the capture substance 90. The capture substance 90 may be suitably selected according to the type of test substance. Examples of useful capture substances 90 include nucleic acids, proteins, peptides, oligosaccharides, antibodies, and nanostructures with specific recognition ability.

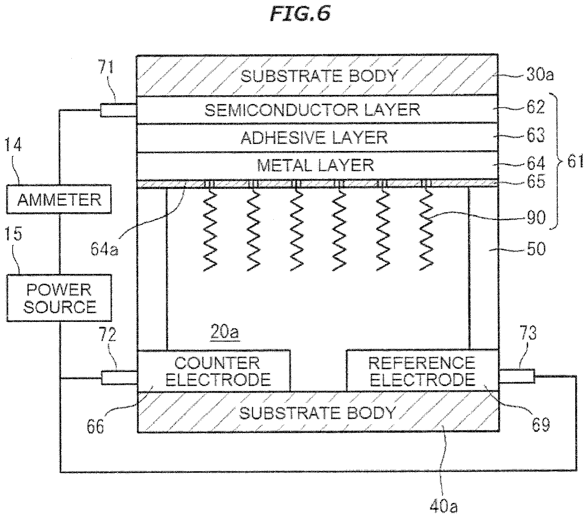

From the perspective of improving detection sensitivity in the present invention, a blocking layer 65 configured by a blocking agent may be formed on part (non-fixed part 64a) of the metal layer 64 where the capture substance 90 is not immobilized, as shown in FIG. 6.

The counter electrode 66 is formed on the substrate man body 40a as shown in FIG. 5. The counter electrode 66 is a thin layer composed of a conductive material. Examples of useful conductive materials include metals such as gold, silver, copper, carbon, platinum, palladium, chromium, aluminum, nickel and the like, or alloys containing at least one thereof, metal oxides such as ATO, FTO, conductive ceramics such as indium oxide and ITO, titanium, titanium compounds such as titanium oxide, titanium nitride and the like. The thickness of the thin film is preferably 1 to 1,000 nm, and more preferably 10 to 200 nm.

The reference electrode 69 is formed on the substrate main body 40a as shown in FIG. 5. The reference electrode 69 is a thin layer composed of a conductive material. Examples of useful conductive materials include metals such as gold, silver, copper, carbon, platinum, palladium, chromium, aluminum, nickel and the like, or alloys containing at least one thereof, metal oxides such as ATO, FTO, conductive ceramics such as indium oxide and ITO, titanium, titanium compounds such as titanium oxide, titanium nitride and the like. The thickness of the thin film is preferably 1 to 1,000 nm, and more preferably 10 to 200 nm. Note that although the reference electrode 69 is provided in the present embodiment, the reference electrode 69 need not necessarily be provided in the present invention. Depending on the type and thickness of the electrode used for the counter electrode 66, the counter electrode 66 also may serve as the reference electrode 69 when measuring a current that has a very slight influence on a voltage drop (for example, 1 µA or less). On the other hand, when measuring a large current, providing the reference electrode 69 is preferable from the perspective of suppressing the voltage drop influence and stabilizing the voltage supplied to the working electrode 61.

The interval holding member 50 is described below. The interval holding member 50 is formed in the shape of a rectangular ring, which is made of silicone rubber insulators. The interval holding member 50 is arranged so as to circumscribe the working electrode 61, counter electrode 66, and reference electrode 69 (refer to FIGS. 4A, 5 and 6). A gap is formed between the top substrate 30 and the bottom substrate 40, and the gap is equivalent to the thickness of the interval holding member 50. Hence, a cavity 20a capable of accommodating sample and electrolyte is formed between the electrodes 61, 66, and 69 (refer to FIGS. 4A, 5 and 6). , The thickness of the interval holding member 50 is usually 0.2 to 300 µm. In the present invention, the material constituting the interval holding member 50 may be, for example, a double-sided plastic tape of polyester film or the like rather than silicone rubber.

In the present invention, the working electrode main body also may be configured by a semiconductor layer and conductive layer. In this case, the electrode lead 71 of the working electrode 61 is connected to the conductive layer. The semiconductor used is not specifically limited, and examples of useful materials include individual semiconductors such as silicon, germanium and the like; oxide semiconductors containing oxides of titanium, tin, zinc, iron, tungsten, zirconium, hafnium, strontium, indium, cerium, yttrium, lanthanum, vanadium, niobium tantalum and the like; perovskite-type semiconductors such as strontium titanate, calcium titanate, sodium titanate, vanadium titanate, potassium niobate and the like; sulfide semiconductors containing sulfides of cadmium, zinc, lead, silver, antimony, bismuth and the like; semiconductors containing nitrides of gallium, titanium and the like; semiconductors formed of selenide of cadmium, lead (for example, cadmium selenide and the like); semiconductors containing cadmium telluride; semiconductors containing phosphide of zinc, gallium, indium, cadmium and the like; semiconductors containing compounds of gallium arsenide, copper - indium - selenide, copper - indium - sulfide and the like; and organic semiconductors or compound semiconductors containing carbon. Note that these semiconductors also may be either true semiconductors or impure semiconductors. Among these examples, oxide semiconductors are preferable. Among true oxide semiconductors, titanium oxide, zinc oxide, tin oxide, niobium oxide, indium oxide, tungsten oxide, tantalum oxide, and strontium titanate are preferable. Among the impure oxide semiconductors, tin-doped indium oxide, and fluorine-doped tin oxide are preferable. The thickness of the semiconductor layer in this case is preferably 0.1 to 100 nm, and more preferably 0.1 to 10 nm.
The conductive layer is formed of an electrically conductive material. Examples of useful conductive materials include metals such as gold, silver, copper, carbon, platinum, paladium, chrome, aluminum, nickel and the like or alloys containing at least one thereof; indium oxide, indium oxide-based materials such as indium oxide containing tin as a dopant; tin oxide, tin oxide-based materials such as tin oxide containing antimony as a dopant (ATO), tin oxide containing fluorine as a dopant (FTO) and the like; titanium, titanium-based materials such as titanium oxide, titanium nitride and the like; and carbon-based materials such as graphite, glassy carbon, pyrolytic graphite, carbon paste, carbon fiber and the like. The thickness of the conductive layer is preferably 1 to 1,000 nm, and more preferably 1 to 200 nm. The thickness of the conductive layer is preferably a thickness ensuring conductivity, that is, providing the least photocurrent (background current) originating from the electrode. Note that the conductive material may be a composite substrate provided with a conductive layer configured by a conductive material disposed on the surface of a nonconductive substrate made of a nonconductive substance such as glass or plastic. The form of the conductive layer may be either a thin film or a spot. Examples of useful materials for constituting the conductive layer include tin-doped indium oxide (ITO), fluorine-doped tin oxide (FTO), antimony-doped tin oxide (ATO) and the like. The conductive layer may be formed by, for example, a film formation method according to the type of material configuring the conductive layer. The film forming method may be the same method as the film forming method used to form the semiconductor layer 62.

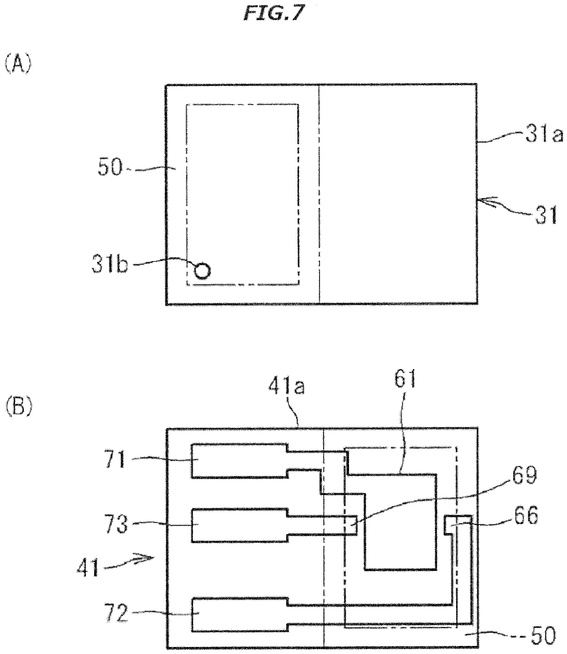

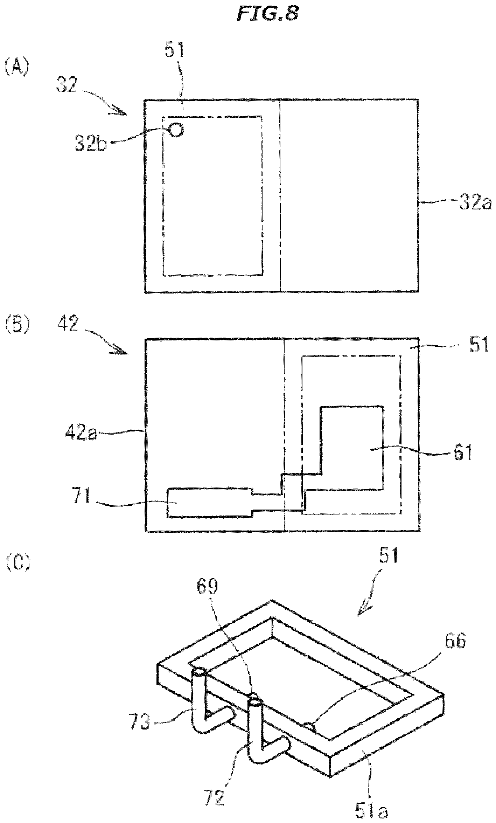

In the present invention, the working electrode 61, counter electrode 66, and reference electrode 69 may be disposed within the frame of the interval holding member 50 so that no electrode comes into contact with another electrode. Therefore, the working electrode 61, counter 66, and reference electrode 69 also may be formed on the same substrate body. That is, the photocurrent detection chip also may have a top substrate 31 constituted by a a sample injection inlet 31 b formed in a substrate body 31 a (refer to FIG. 7A), and a bottom substrate 41 constituted by a working electrode 61, counter electrode 66, and reference electrode 69 formed on a substrate main body 41 a (corresponding to the working electrode substrate of the present embodiment) (refer to FIG. 7B). In the present invention, the counter electrode 66 and the reference electrode 69 need not be thin film electrodes formed on a substrate body. That is, the photocurrent detection chip may have a top substrate 32 (refer to FIG. 8A) wherein a sample injection inlet 32b is formed on a substrate body 32a, a bottom substrate 42 (refer to FIG. 8B) wherein the working electrode 61 is formed on the substrate body 42a (corresponding to the working electrode substrate in the present embodiment), and an interval holding member 51 wherein the counter electrode 66 and the reference electrode 69 are provided on a member body 51a (refer to FIG. 8C). In this case, at least either the counter electrode 66 or the reference electrode 69 is provided on the member body 51a of the interval holding member 51. The other electrodes, except those electrodes provided on the member body 51a, may be provided on either of the top substrate 32 and bottom substrate 42.

Note that in the present invention the material constituting the substrate that is not provided with the working electrode may be a material which is permeable to light.

### [Method of Manufacturing Electrodes]

The electrode manufacturing method of the present invention is a method of manufacturing a photocurrent detection electrode used for photoelectrochemically detecting a test substance from which electrons originate via photoexcitation, and includes
(A) a step of forming an adhesive layer containing linker molecules on an electrode body configured by a semiconductor which accepts the electrons produced via photoexcitation, and
(B) a step of forming a metal layer on the adhesive layer.

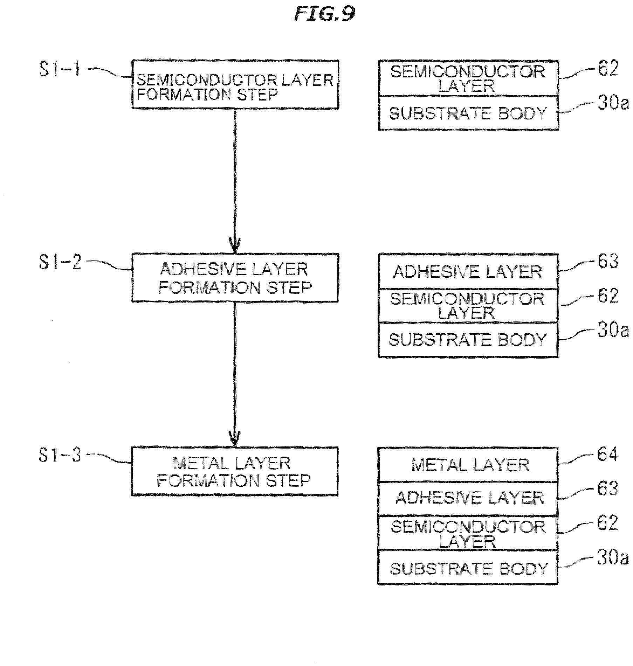

FIG. 9 is a process chart showing the processing sequence of an embodiment of the electrode manufacturing method of the present invention. In the electrode manufacturing method of the present embodiment, a semiconductor layer 62 is first formed on a substrate main body 30a as a working electrode body (step S1-1). In step S1-1, the semiconductor layer 62 is formed by a thin film forming method according to the type of semiconductor constituting the semiconductor layer 62. Deposition method, sputtering, imprinting, screen printing method, plating method, sol-gel method, spin coating, dipping, vapor deposition and the like may be used as a film forming method.

The adhesive layer 63 is then formed as a working electrode body on the semiconductor layer 62. (step 1-2). In step S1-2, the adhesive layer 63 may be formed by a method wherein the substrate body bearing the semiconductor layer formed as a working electrode body is immersed in a solution containing a linker molecule, an imprinting method, screen printing method, spin coat method, vapor deposition method, spray method, dip coating method and the like. In the electrode manufacturing method of the present embodiment, the adhesive layer formed on the semiconductor layer 62 provides stronger adhesion between semiconductor layer acting as the working electrode body, and the metal layer 64 which is formed in the following step. As a result, the photocurrent detection chip which has electrodes obtained by the electrode manufacturing method of the present embodiment is capable of detecting a sample substance with a high degree of reproducibility as previously mentioned.

In the electrode manufacturing method of the present embodiment, a metal layer 64 is formed on the adhesive layer 63 (step S1-2). In step S1-2, the metal layer 64 is formed, for example, by a thin film forming method according to the type of metal. Examples of usable thin film forming methods include vapor deposition, spattering, imprinting, screen printing, plating, and sol-gel methods.

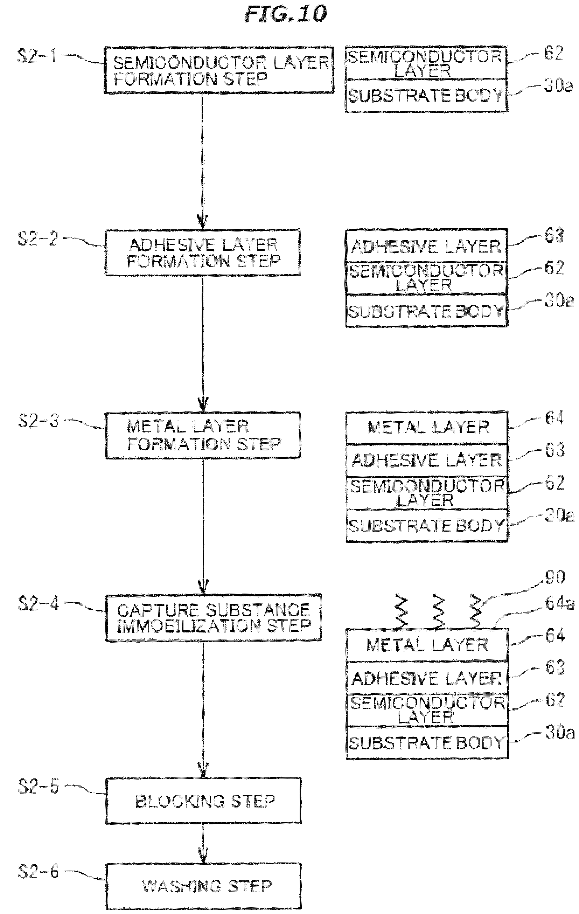

FIG. 10 is a process chart showing the processing sequence of an embodiment of the electrode manufacturing method of the present invention. In the electrode manufacturing method of the present embodiment, a capture substance 90 is immobilized on the surface of the metal layer 64, and a blocking layer 65 is formed on the part of the metal layer 64 wherein the capture substance 90 is not present (nonfixed part 64a) (refer to FIG. 6). In the electrode manufacturing method of the present embodiment, steps 2-1 and 2-2 are performed via operations which are identical to those of steps S1-1 and 1-2.

After step S2-3 in the electrode manufacturing method of the present embodiment, the capture substance 90 which captures the test substance is immobilized on the metal layer 64 (step S2-4). Immobilizing the capture substance 90 on the metal layer 64 can be accomplished via a linking group which binds to the metal layer 64. Usable linkage groups may include, for example, a thiol group, hydroxyl group, a phosphate group, a carboxyl group, carbonyl group, aldehyde, sulfonic acid, an amino group and the like. Immobilizing the capture substance 90 on the surface of the metal layer 64 also may be accomplished via a photosetting resin, physical adsorption or the like. The amount of immobilization of the capture substance 90 on the metal layer 64 is not specifically limited and may be set according to the use and purpose.

Thereafter, the part of the metal layer 64 on which the capture substance 90 is not present (non-fixed part 64a) is blocked by a blocking agent (step S2-5). In step S2-5, the blocking agent of the non-fixed part 64a is accomplished via a method wherein a blocking agent is brought into contact with the non-fixed part 64a, imprinting method, screen printing method, spin coating method, vapor deposition method, spray method, dipping method or the like. The blocking agent will prevent nonspecific adhesion of substances other than the test substance on the non-fixed part 64a. Examples of useful blocking agent include compounds such as triethylene glycol mono-11 - mercaptoundecanoic decyl ether, 1 - mercapto hexanol, 2 - mercaptoethanol, mercaptoethanol, phosphine [bis (p-sodium sulfonate) phenyl], bovine serum albumin (BSA) (for example, 1 to 10wt% concentration used), human serum albumin (HSA) (e.g., 1 to 10wt% concentration used), skim milk powder (for example, 1 to 10 wt% concentration used), casein (e.g., 1 to 10wt% concentration used), gelatin (e.g., 1 to 10 wt% concentration used), protein that does not bind to the test substance or the sample substance, nucleic acid that does not bind to the sample substance or the test substance, surface active agents (e.g., Tween20, Triton X-100, SDS) and the like.ike. The blocking agent also may be a solution wherein these compounds and substances are dissolved in a solvent such as water buffer solution or the like. In this case, the concentration of the compound in the solution may be suitably set according to the type of compound. Examples of blocking agent in the present invention include commercial blocking agents such as NanoBioblocker (trademark) and the like.

After step S2-5, the substrate body is washed (step S2-6). A substrate which has a working electrode (working electrode substrate) is thus obtained. In step S2-6, a washing agent is used according to the type of capture substance when washing the substrate body. For example, if the capture substance is a nucleic acid, the washing agent is a buffer solution, or hybridization solution is used in hybridizing the nucleic acid when the capture substance is a protein, the washing agent used is a buffer solution or the like. Washing the substrate body is accomplished by a method wherein the substrate body is immersed in the washing agent, or a method wherein the surface of the substrate body is rinsed with a flow of the washing agent. The washing step also may be performed as necessary after step S2-4.

According to the present invention as described above, the electrode (working electrode) can be obtained by simple operations.

### [Method of Detecting a Sample Substance]

The method of detecting a sample substance using the electrode (working electrode 61) of the present embodiment of the invention is described below.

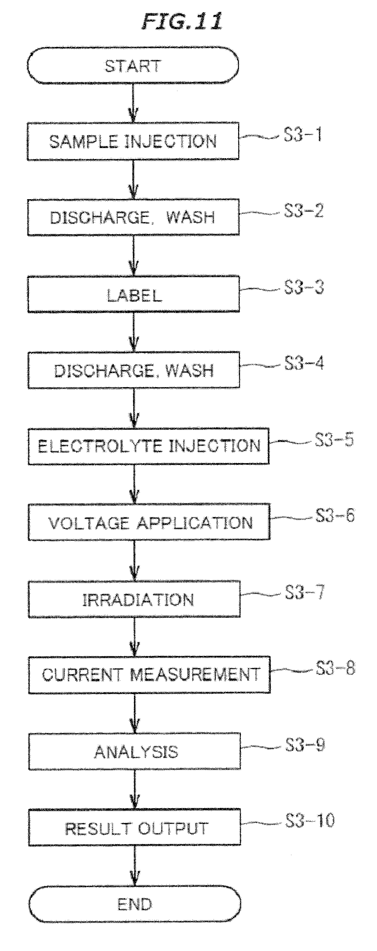

FIG. 11 is a process chart showing the processing sequence of an embodiment of the sample substance detection method using the electrode of the present invention.

In step S3-1 shown in FIG. 11, the user injects a liquid sample containing the sample substance via the sample injection inlet 30b of the photocurrent detection chip 20. The sample substance in the liquid sample is captured by the capture substance 90 on the top substrate 30 in the working electrode 61 configuring the photocurrent detection chip 20.

The capture substance 90 may be suitably selected according to the type of sample substance. For example, if the sample substance is a nucleic acid, the capture substance 90 may be a nucleic acid antibody or a nucleic acid probe which hybridizes to the nucleic acid. When the sample substance is a ligand, the capture substance 90 may be a receptor for that ligand. When the sample substance is a receptor, the capture substance may be a ligand for that receptor.

Capturing the sample substance with the capture substance 90 can be carried out, for example, under the condition that the sample substance is bound to the capture substance 90. The condition of binding the sample substance to the capture substance 90 can be suitably selected according to the type of sample substance. For example, when the sample substance is a nucleic acid and the capture substance 90 is a nucleic acid probe which hybridizes to the nucleic acid, capturing the sample substance can be carried out in the presence of a hybridization buffer solution. When the sample substance is a nucleic acid or peptide and the capture substance 90 is an antibody of that nucleic acid or an antibody for that peptide, capturing the sample substance can be conducted via and antigen-antibody reaction in a suitable solution such as phosphate-buffered saline (PBS), HEPES buffer, PIPES buffer, Tris buffer and the like. When the sample substance is a ligand and the capture substance 90 is a receptor for that ligand, or when the sample substance is a receptor and the capture substance 90 is a ligand for that receptor, capturing the sample substance can be conducted in a suitable solution to bind the ligand and receptor.

In this sample substance detection method, peeling of the metal layer 64 bearing the immobilized capture substance 90 for capturing the sample substance can be suppressed in step S3-1 by using the working electrode which has the adhesive layer 63. Detection of the sample substance is therefore performed with a high degree of reproducibility.

In step S3-2, the user then discharges the residual liquid containing the contaminants from the sample injection inlet 30b of the photocurrent detection chip 20, and washes the interior of the photocurrent detection chip 20. Noise generation caused by the contaminants is thus eliminated. In step S3-2, washing of the interior of the photocurrent detection chip 20 may be accomplished using, for example, a buffer solution (especially a buffer solution containing surface active agent), pure water (especially pure water containing surface active agent), or an organic solvent such as ethanol.

In step S3-3, the user then injects a liquid containing a labeled binder substance (test substance) containing a labeled substance and a binder for binding to the sample substance into the sample injection inlet 30b of the photocurrent detection chip 20. Hence, the labeled binder bonds to the sample substance captured on the working electrode 61, and the sample substance is thereby labeled.

The labeled binder is constituted by a labeled substance and a binder for binding to the sample substance. The labeled substance releases electrons which are excited via irradiation with light. The labeled substance may be at least one item selected from groups of metal complexes, organic phosphors, quantum dots, and inorganic phosphors. [fuzzy]Examples of labeled substances include metal phenolphthalein, ruthenium, osmium complex, iron complex, zinc complex, 9-phenyl-xanthene dyes, cyanine dyes, cyanine metalloproteinases, xanthene dyes, triphenylmethane dyes, acridine dyes, oxazine dyes, coumarin dyes, merocyanine dyes, rhoda-cyanine dyes, polymethine dyes, porphyrin dyes, phthalocyanine dyes, rhodamine dyes, xanthene dyes, chlorophyll pigments, eosin dyes, mercurochrome dyes, indigo dyes, BODIPY dyes, CALFluor dyes, Oregon Green dyes, Rhodol Green, Texas Red, Cascade Blue, nucleic acids (DNA, RNA and the like), cadmium selenide, cadmium telluride, Ln203:Re, ZnO, CaWO4, MO-xAl203:Eu, Zn2SiO4:Mn, LaPO4: Ce, b, Cy3, Cy3.5, Cy5, Cy5.5, Cy7, Cy7.5, and Cy 9 (manufactured by Amersham Biosciences); Alexa Fluor 355, Alexa Fluor 405, Alexa Fluor 430, Alexa Fluor 488, Alexa Fluor 532, Alexa Fluor 546, Alexa Fluor 555, Alexa Fluor 568, Alexa Fluor 594, Alexa Fluor 633, Alexa Fluor 647, Alexa Fluor 660, Alexa Fluor 680, Alexa Fluor 700, Alexa Fluor 750, and Alexa Fluor 790 (manufactured by Molecular Probes); DY-610, DY-615, Dry-630, DY-631, DY-633, DY-635, DY-63 6, EVOb lue 10, EVOblue 30, DY-647, DY-650, D Y-65 1 , DY-800, DYQ-660 and DYQ-661 (manufactured by Dyomics); At to 425 , At to 465, At to 488, At to 495, At to 520, At to 532, At to 550, At to 565, A**t** to 590, At to 594, At to 610, At to 611 X, At to 620 , At to 633, At to 635, At to 637, At to 647, At to 655, At to 680, At to 700, At to 725, At to 740 (manufactured by At to - TEC GmbH); Vivo Tag S680, Vivo Tag 680 and Vivo Tag S750 (manufactured by Vis En Medical) and the like. Note that Ln represents La, Gd, Lu, or Y; Re represents a lanthanide element, M represents an alkaline earth metal element, and x represents an integer from 0.5 to 1.5. For other examples of labeled substances refer to U.S. Patent Publication No. 2009/0294305, Japanese Patent Publication No. 7-83927, and Japanese Patent Publication No. 2008-154179.

The binder may be suitably selected according to the type of sample substance insofar as the binder will bond to the capture substance 90 in the sample substance. For example, if the sample substance is a nucleic acid, the binder may be a nucleic acid antibody or a nucleic acid probe which hybridizes to the nucleic acid. When the sample substance is a protein or peptide, the binder may be an antibody of the protein or peptide. When the sample substance is a ligand, the binder may be a receptor for that ligand. When the sample substance is a receptor, the binder may be a ligand for that receptor.

In step S3-3, the residual labeled binder that has not bonded to the sample substance is present in a free state in the liquid within the photocurrent detection chip 20. In step S3-4, the user discharges the liquid from the sample injection inlet 30b of the photocurrent detection chip 20, and washes the interior of the photocurrent detection chip 20. In this way the characteristics of the detection results are improved. Note that the washing step need not be carried out. In step S3-4, for example, a buffer solution (especially a buffer solution containing surface active agent), pure water (especially pure water containing surface active agent), or an organic solvent such as ethanol may be used.

In step S3-5, the user then injects electrolyte through the sample injection inlet 30b of the photocurrent detection chip 20. A solution containing an electrolytic substance consisting of a salt capable of supplying electrons to the labeled substance, an aprotic polar solvent, protic polar solvent or a mixture of a protic polar solvent and aprotic polar solvent may be used as the electrolyte solution. The electrolyte also may contain other constituent ingredients as desired. The electrolyte also may be a gel or solid.

Examples of materials useful as the electrolyte include iodide, bromide, metal complexes, thiosulfate, sulfite, and mixtures thereof. Specific examples of the electrolytic substances include metal iodides such as lithium iodide, sodium iodide, potassium iodide, cesium iodide, and calcium iodide; iodized salts of quaternary ammonium compounds such as tetraalkylammonium iodide, pyridinium iodide, imidazolium iodide and the like; metal bromides such as lithium bromide, sodium bromide, potassium bromide, cesium bromide, and calcium bromide; bromide salts of quaternary ammonium componds such as tetraalkylammonium bromide, and pyridinium bromide; metal complexes such as ferrocyanide salts, and ferricyanide ions; thiosulfates such as sodium thiosulfate, ammonium thiosulfate, potassium thiosulfate, and calcium thiosulfate; sulfites such as sodium sulfite, potassium sulfite, ammonium sulfite, iron sulfite, sodium bisulfite, and calcium sulfite; and mixtures thereof. Among these, tetrapropylammonium iodide and calcium iodide are preferable. The concentration of electrolytic substance in the electrolyte solution is preferably 0.001 to 15M.

Water, and polar solvent composed mainly of a mixture of water and liquid buffering component may be used as the protic polar solvent. Aprotic polar solvents include nitriles such as acetonitrile (CH₃ CN); carbonates such as propylene carbonate and ethylene carbonate, heterocyclic compounds such as 1,3 - dimethyl-imidazolinone, 3 - methyl-non-oxazolinyl,and dialkyl imidazolium salts; dimethyl formamide, dimethyl sulfoxide, sulfolane and the like. Among these aprotic polar solvents, acetonitrile is preferred. Aprotic polar solvents and protic polar solvents can be used individually or as a mixture thereof. The mixture of polar aprotic polar solvent and protic polar solvent is preferably a mixture of water and acetonitrile.

When the electrolytic substance is iodide, the metal constituting the metal layer 64, preferably gold, is dissolved via the injection of the electrolyte. At this time, the capture substance 90, the sample substance, and the labeled binder (test substance) are present on the working electrode 61, and electrons are transferred between the semiconductor 62 of the working electrode 61, and the labeled substance in the labeled binder.

An electrolytic solution containing iodine or iodide is preferable as the electrolyte.

After the user performs steps S3-1 through S3-5, the user inserts the photocurrent detection chip 20 into the chip receiver 11 of the detection device 1 shown in FIG. 1. The user then instructs the detection device 1 to begin measurements. The electrode leads 71, 72, and 73 of the photocurrent detection chip 20 inserted into the detection device 1 are connected to the ammeter 14 and power source 15. An optional electric potential is supplied from the power source 15 of the detection device 1 to the working electrode 61 based on the reference electrode 69 (step S3-6). It is preferred that the potential supplied to the electrode is less than the current value (steady-state current, dark current) when excitation light irradiates the test substance to maximize the photocurrent originating from the test substance. The potential may be supplied to the counter electrode or the working electrode.

In step S3-7, excitation light irradiates the labeled substance on the working electrode 61 from the light source 13 of the detection device 1. The labeled substance is thus excited and releases electrodes. The released electrons migrate to the semiconductor layer 62. As a result, a current flows between the working electrode 61 and the counter electrode 66. Note that in this step, the labeled substance also may be irradiated only with light of a specific wavelength using a splitter and bandpass filter as necessary.

In step S3-8, the ammeter 14 of the detection device 1 measures the current flowing between the working electrode 61 and the counter electrode 66. The current value measured by the ammeter 14 correlates to the number of objects of the labeled substance. The sample substance therefore can be quantified based on the measured current value.

In step S3-9, the current value is digitally converted by the A/D converter 16 and the result is input to the controller 17. The controller 17 then estimates the amount of sample substance in the liquid sample from the digitally converted current value based on a previously created calibration curve describing the relationship between the amount of sample substance and the current value. The controller 17 then prepares a detection result screen for displaying the information of the estimated amount of sample substance on the display 12.

In step S3-10, the detection result screen prepared by the controller 17 is transmitted to the display 12 and displayed on the display 12.

In the sample substance detection method using the electrode (working electrode 61) of the embodiment of the present invention as described above, peeling of the metal layer 64 is suppressed when the capture substance 90 captures the sample substance in step S3-1. Therefore, the sample substance can be detected with a high degree of reproducibility by suppressing fluctuation of the amount of capture substance 90 on the working electrode 61 due to peeling of the metal layer 64.

### [Examples]

Although the present invention is described in detail below by way of examples, the present invention is not limited to these examples.

### (Fabrication Example 1)

Solution A was produced by adding 3-amino propyl triethoxysilane (APTES), which is a silane coupling agent, to toluene to achieve a concentration of 1vol%.

### (Fabrication Example 2)

Solution B was produced by adding 3 - mercaptopropyl triethoxysilane (MPTES), which is a silane coupling agent, to toluene to achieve a concentration of 1vol%.

### (Fabrication Example 3)

Thiolated DNA was obtained by introducing thiol group to a DNA strand of 24 nucleotides. The obtained thiolated DNA was added to sterile purified water to a concentration of 1 µM to produce an aqueous DNA solution.

### (Fabrication Examples 4 through 6)

An SH-TEG aqueous solution (fabrication example 4) was obtained by adding triethylene glycol mono-11 - mercaptoundecanoic decyl ether (SH-TEG) to a TBS buffer solution (25mM trishydroxymethyl aminomethane, 0.15 M sodium chloride, pH 7.4) to a concentration of 1 mM. An MCH aqueous solution (fabrication example 5) was obtained by adding mercapto hexanol (MCH) to TBS buffer to a concentration of 1 mM. A commercial blocking agent (NanoBioblocker (trademark), manufactured by NanoBioTech Company) was used as the blocking agent of fabrication example 6.

### (Reference Example 1)

### (1) Forming the Working Electrode Body

A semiconductor layer (working electrode body) constituted by a thin film (approximately 200 nm thickness) of tin-doped indium oxide was formed on a substrate body of silicon dioxide (SiO₂) via a spattering method. A working electrode lead for connecting to the ammeter was then connected to the semiconductor layer to obtain a substrate a1. The semiconductor layer performs as both a conductive layer and an electron acceptor layer.

### (2) Forming the Metal Layer

A metal layer constituted by a thin gold film (approximately 2 nm thickness) was formed on the semiconductor layer formed in example (1) via vacuum vapor deposition to obtain a substrate a2.

### (3) Immobilizing the Capture Substance

7 µL of the aqueous DNA solution obtained in fabrication example 3 was titrated on the metal layer of the substrate a2 obtained in example (2). Silicone rubber (0.1 mm thickness) was then disposed as a wall around the perimeter of the metal layer of the substrate a2. Thereafter, a cover glass was set on the silicone rubber to seal the cavity circumscribed by the substrate a2 and the silicone rubber. The substrate a2 was then allowed to stand overnight at 4°C to covalently bond the gold constituting the thin gold film and the thiolated DNA constituting the capture substance. The metal layer of the substrate a2 was thereafter washed with TBS buffer solution. A substrate a3 was thus obtained.

### (4) Blocking Process

Silicone rubber (0.1 mm thickness) was disposed as a wall around the perimeter of the metal layer of the substrate a3. 7 µL of the blocking agent obtained in fabrication example 4 was subsequently titrated in the cavity circumscribed by the substrate a3 and the silicone rubber. Then, a cover glass was set on the silicone rubber to seal the cavity circumscribed by the substrate a3 and the silicone rubber. The substrate a3 was allowed to stand overnight at 4°C to block the part of the metal layer in which the capturer substance was not immobilized. The metal layer of the substrate a3 was thereafter washed with TBS buffer solution. A substrate a4 was thus obtained.

### (5) Washing

The following washing operation was performed to remove the thiolated DNA which was not immobilized on the metal layer. Silicone rubber (0.1 mm thickness) was disposed as a wall around the periphery of the metal layer of the substrate a4, and the entirety was placed in a hybridization chamber (20 µL volume) made of fluororesin (teflon (trademark)). 20 µL of hybridization solution (PerfectHyb (trademark) Hybridization solution, manufactured by Toyobo Company, Ltd.) was injected into the cavity formed by the hybridization chamber. The substrate a4 was then allowed to stand for 2 hours in a thermoregulated bath (65°C). The liquid was subsequently discharged from the cavity. 20 µL of hybridization aqueous solution was then injected into the cavity and subsequently discharged from the cavity. The produced working electrode substrate was then removed from the hybridization chamber. In this way, a working electrode substrate, which has the hybridization chamber, was obtained.

### (Experiment 1)

### (1) Hybridization Process

20 µL of hybridization solution was injected into the hybridization chamber which was mounted on the working electrode substrate obtained in reference example 1. The working electrode substrate was then allowed to stand 2.5 hours in a thermoregulated bath (65°C). The hybridization chamber was subsequently removed upward from the electrode. The working electrode substrate was washed three times with 0.5 mL washing solution (2xSCC solution containing 0.01 wt% SDS), then washed with 0.5 mL sterile purified water and subsequently dried.

### (2) Metal Layer Stability Evaluation

The stability of the metal layer on the working electrode body (semiconductor layer) after (1) was visually evaluated. This visual inspection revealed that the metal layer has completely peeled away. If there is a peeling of the metal layer when the sample substance is captured by hybridization, the metal layer together with the capture substance on the metal layer is missing from the working electrode body. This situation will reduce the reproducibility of detection sensitivity and measurements when a sample substance has been captured using the working electrode substrate (Reference Example 1) which has the metal layer formed on the semiconductor layer.

### (Example 1)

### (1) Forming the Working Electrode Body

A substrate A1 was obtained by forming a semiconductor layer (working electrode body) constituted by a thin film (approximately 200 nm thickness) of tin-doped indium oxide on a substrate body of silicon dioxide (SiO₂) via a spattering method, and adding a working electrode lead thereon for connecting the working electrode body with the ammeter. This thin film performed the functions of both a conductive layer and an electron acceptor layer.

### (2) Forming the Adhesive Layer

The substrate A1 obtained in step (1) was immersed for 1 hour in the solution obtained in fabrication example 1. The substrate A1 was subsequently washed in toluene, and dried. An adhesive layer constituted by a thin film of APTES was thus formed on the semiconductor layer.

### (3) Forming the Metal Layer

A metal layer constituted by a thin gold film (approximately 2 nm thickness) was formed on the adhesive layer formed in step (2) via vacuum vapor deposition to obtain a substrate A2.

### (4) Immobilizing the Capture Substance

7 µL of the aqueous DNA solution obtained in fabrication example 3 was titrated on the metal layer of the substrate A2 obtained in step (3). Silicone rubber (0.1 mm thickness) was then disposed as a wall around the perimeter of the metal layer of the substrate A2. Thereafter, a cover glass was set on the silicone rubber to seal the cavity circumscribed by the substrate A2 and the silicone rubber. The substrate A2 was then allowed to stand overnight at 4°C to covalently bond the gold constituting the thin gold film and the thiolated DNA constituting the capture substance. The metal layer of the substrate A2 was thereafter washed with TBS buffer solution. A substrate A3 was thus obtained.

### (5) Blocking Process

Silicone rubber (0.1 mm thickness) was disposed as a wall around the perimeter of the metal layer of the substrate A3. 7 µL of the blocking agent obtained in fabrication example 5 was subsequently titrated in the cavity circumscribed by the substrate A3 and the silicone rubber. Then, a cover glass was set on the silicone rubber to seal the cavity circumscribed by the substrate A3 and the silicone rubber. The substrate A3 was allowed to stand overnight at 4°C to block the part of the metal layer in which the capturer substance was not immobilized. The metal layer of the substrate A3 was thereafter washed with TBS buffer solution. A substrate A4 was thus obtained.

### (6) Washing

The following washing operation was performed to remove the thiolated DNA which was not immobilized on the metal layer. Silicone rubber (0.1 mm thickness) was disposed as a wall around the perimeter of the metal layer of the substrate A4. A hybridization chamber (20 µL volume) was then placed on the silicone rubber. 20 µL of hybridization aqueous solution was injected into the cavity formed by the hybridization chamber. The substrate A4 was then allowed to stand for 2 hours in a thermoregulated bath (65°C). The liquid was subsequently discharged from the cavity. 20 µL of hybridization aqueous solution was then injected into the cavity and subsequently discharged from the cavity. In this way a working electrode substrate which has the hybridization chamber was obtained.

### (Example 2)

A working electrode substrate was produced using identical operations to those of Example 1, with the exception that the blocking agent of fabrication example 6 was used rather than the blocking agent obtained in fabrication example 5 in Example 1.

### (Example 3)

A working electrode substrate was produced using identical operations to those of Example 1, with the exception that the blocking process of Example 1 was not performed.

### (Example 4)

A working electrode substrate was produced using identical operations to those of Example 1, with the exception that the solution B of fabrication example 2 was used rather than the solution A obtained in fabrication example 1 in Example 1.

### (Example 5)

A working electrode substrate was produced using identical operations to those of Example 4, with the exception that the blocking agent of fabrication example 6 was used rather than the blocking agent obtained in fabrication example 5 in Example 4.

### (Example 6)

A working electrode substrate was produced using identical operations to those of Example 4, with the exception that the blocking process of Example 4 was not performed.

### (Comparative Example 1)

A working electrode substrate was produced using identical operations to those of reference Example 1, with the exception that the blocking agent of fabrication Example 5 was used rather than the blocking agent obtained in fabrication Example 4 in reference example 1.

### (Comparative Example 2)

A working electrode substrate was produced using identical operations to those of reference Example 1, with the exception that the blocking agent of fabrication Example 6 was used rather than the blocking agent obtained in fabrication Example 4 in reference example 1.

### (Comparative Example 3)

A working electrode substrate was produced using identical operations to those of reference Example 1, with the exception that the blocking process of reference Example 1 was not performed.

### (Experiment 2)

Stability was evaluated for the metal layer on the working electrode on the working electrode substrates obtained in Examples 1 through 6 and comparative Examples 1 through 3 by performing identical operations to those of experiment 1. Evaluation preparations are described below. Evaluation results are shown in Table 1.

### [Evaluation Preparation]

o indicates the metal layer remains completely.
x indicates peeling of the metal layer is observed.

**Table 1**

| | linker molecule | Blocking agent | Evaluation |
|---|---|---|---|
| Ex. 1 | APTES | A | ○ |
| Ex.2 | APTES | B | ○ |
| Ex. 3 | APTES | - | ○ |
| Ex. 4 | MPTES | A | ○ |
| Ex. 5 | MPTES | B | ○ |
| Ex. 6 | MPTES | - | ○ |
| Comp Ex. 1 | - | A | × |
| Comp. Ex. 2 | - | B | × |
| Comp. Ex. 3 | - | - | × |

It can be understood from the results shown in Table 1 that the metal layer was maintained satisfactorily without any observable peeling of the metal layer even in the liquid phase used in hybridization between amino acids when the adhesive layer was interposed between the metal layer and the semiconductor layer (examples 1 through 6). These results also therefore suggest that a sample substance can be detected with high degrees of detection sensitivity and reproducibility when the detection of the sample substance is performed using the working electrode substrates obtained in examples 1 through 6. In examples 1 through 6, the silane coupling agent used in the adhesive layer is a compound that binds to both the semiconductor layer and the metal layer. It is therefore suggested that if a photocurrent detection electrode, which has an adhesive layer constituted by a compound that binds to both the semiconductor layer and the metal layer, is disposed between metal layer and semiconductor layer, a sample substance can be detected with high degrees of detection sensitivity and reproducibility by preventing separation of the metal layer.

On the other hand, it can also be understood from the results in Table 1 that peeling of the metal layer was observed when the adhesive layer was not formed between the semiconductor layer and the metal layer (comparative examples 1 through 3). Low degrees of detection sensitivity and reproducibility, therefore, can be assumed from these results when detecting a sample substance using the working electrode substrates obtained in comparative examples 1 through 3.

### (Fabrication Example 7)

20 µL of FITC-labeled goat anti-mouse immunoglobulin antibody solution (Dako Company, catalog No. F0479) was mixed with 20 µL of tris (2 - chloroethyl) phosphate (TCEP) (product of Pierce Company, catalog No. 77712), a reducing agent. The obtained mixture was agitated for 90 minutes at room temperature and 1800 rpm (revolutions/minute) to obtain Fab fragments. Thereafter, 6 µL of the supernatant of the above mixture and 594 µL buffer solution were mixed to obtain a Fab antibody solution.

### (Example 7)

### (1) Forming the Working Electrode Body

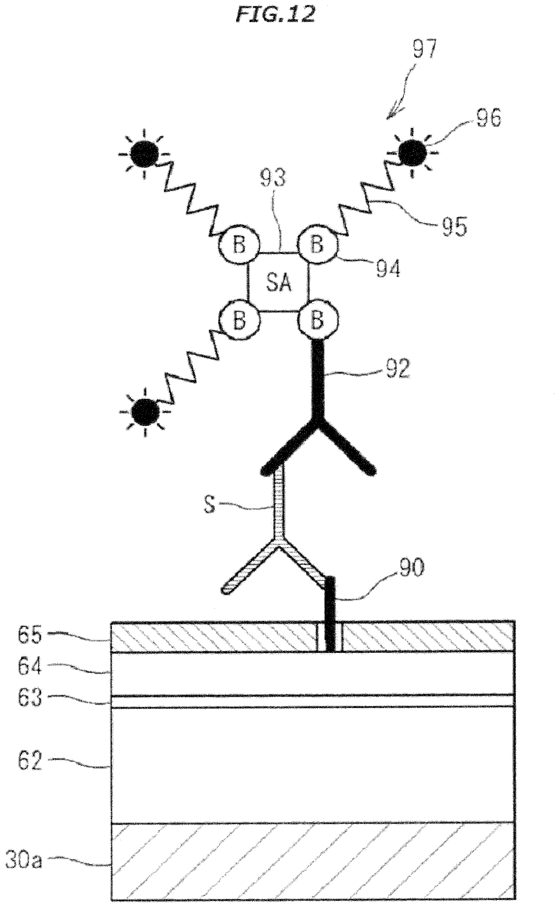

A substrate A1 was obtained by forming a semiconductor layer 62 (working electrode body; refer to FIG. 12) constituted by a thin film (approximately 200 nm thickness) of tin-doped indium oxide on a substrate body of silicon dioxide (SiO₂) via a spattering method, and adding a working electrode lead thereon for connecting the working electrode body with the ammeter. The semiconductor layer 62 performs as both a conductive layer and an electron acceptor layer.

### (2)Adhesive Layer Formation

The substrate A1 obtained in step (1) was immersed for 1 hour in the solution obtained in fabrication example 1. The substrate A1 was subsequently washed in toluene, and dried. An adhesive layer 63 constituted by a thin layer af APTES silane coupling agent was thus formed on the semiconductor layer 62.

### (3) Forming the Metal Layer

A metal layer 64 constituted by a thin gold film (approximately 2 nm thickness) was formed on the adhesive layer 63 formed in step (2) via vacuum vapor deposition to obtain a substrate A2.

### (4) Immobilizing the Capture Substance

Silicone rubber (0.1 mm thickness) was then disposed as a wall around the perimeter of the metal layer 64 of the substrate A2. A chamber (20 µL volume) was then placed on the silicone rubber. 20 µL of the Fab antibody solution obtained in fabrication example 7 was injected into the cavity formed by the chamber. The substrate A2 was then allowed to stand overnight at 4°C. The gold constituting the metal layer 64 and the capture substance 90 (Fab antibody) were thus covalently bonded. Thereafter, the metal layer 64 of the substrate A2 was washed with 20 µL TBS buffer while the chamber remained in place. A substrate A3 was thus obtained.

### (5) Blocking Process

Silicone rubber (0.1 mm thickness) was disposed as a wall around the perimeter of the metal layer 64 of the substrate A3. 7 µL of the blocking agent obtained in fabrication example 5 was subsequently titrated in the cavity circumscribed by the substrate A3 and the silicone rubber. Then, a cover glass was set on the silicone rubber to seal the cavity circumscribed by the substrate A3 and the silicone rubber. The substrate A3 was then allowed to stand overnight at 4°C to form a blocking layer 65 (refer to FIG. 12) on the partial surface of the metal layer 64 where the capture substance 90 (Fab antibody) was not immobilized. The metal layer 64 of the substrate A3 was thereafter washed with TBS buffer solution. In this way a working electrode substrate was obtained.

### (Fabrication Examples 8 through 11)

Mouse immunoglobulin used as a sample substance S was added in concentrations of 0.1 ng/mL (fabrication example 8), 1 ng/mL (fabrication example 9), 10 ng/mL (fabrication example 10), and 100 ng/mL (fabrication example 11) to tris buffered physiological saline solution (TBS-T; TBS containing 0.1 vol% polyoxyethylene sorbitan monolaurate (Tween-20), pH 7.4) containing 1 wt% bovine serum albumin, to obtain a sample liquid containing the sample substance.

### (Fabrication Example 12)

Biotin 94 and photochemically active labeled substance 96 (AlexaFluor750) was introduced to DNA 95 which is 24 nucleotides in length, to obtain a biotinylated labeled DNA 97. The obtained biotinylated DNA 97 was added to TBS-T to achieve a concentration of 100 nM, and a labeled solution was thus obtained.

### (Fabrication Example 13)

Acetonitrile and ethylene carbonate were mixed at a volume ratio of 2:3 to obtain an apriotic polar solvent. Tetrapropylammonium iodide was added as an electrolytic salt to the apriotic polar solvent to obtain a concentration of 0.6 M. Then, iodine was dissolved as a further electrolyte in the above obtained liquid to achieve a concentration of 0.06 M and obtain a dissolved attractant electrolyte. Note that the iodine and tetrapropylammonium iodide can dissolve the gold.

### (Fabrication Example 14)

A platinum thin film counter electrode (200 nm thickness) a platinum thin film reference electrode (200 nm thickness), and electrode leads for connecting the counter electrode and reference electrode with the ammeter were formed on a substrate body of silicon dioxide (SiO₂) via a spattering method. In this way a counter electrode substrate was obtained.

### (Experiment 3)

### (1) Capturing the Sample substance

20 µL of the sample liquid obtained in fabrication examples 8 through 11 were respectively injected into the chamber in which the working electrode substrate obtained in example 7 was mounted. The working electrode substrate was allowed to stand for 1.5 hours at room temperature, and the sample substance was captured by capture substance 90 (Fab antibody) (refer to FIG. 12). Thereafter, the working electrode was washed with 20 µL of TBS-T while the chamber remained in place.

### (2) Labeling

Biotin labeled anti mouse immunoglobulin antibody (biotinylated secondary antibody) solution (Sigma Aldrich Company, catalog No. B7151) was diluted with TBS-T containing 1 wt% bovine serum albumin to 2000 times volume to obtain a dilute antibody solution. 20 µL of the obtained dilute antibody solution was injected into the chamber of the working electrode substrate after step (1). The working electrode substrate was allowed to rest for 30 minutes at room temperature to produce a reaction between the biotin labeled secondary antibody 92, and the sample substance S (refer to FIG. 12). Thereafter, the working electrode was washed with 20 µL of TBS-T while the chamber remained in place.

A streptavidin-containing solution (Vector Company, product No. SA-5000) was diluted with TBS-T to 500 times volume to obtain a streptavidin-containing dilute solution. 20 µL of the obtained streptavidin-containing solution was injected into the chamber of the working electrode substrate. The working electrode substrate was allowed to rest for 30 minutes at room temperature to produce a reaction between the streptavidin 93 and the biotin labeled secondary antibody 92 present on the working electrode (refer to FIG. 12). Thereafter, the working electrode was washed with 20 µL of TBS-T while the chamber remained in place.

20 µL of the labeled solution obtained in fabrication example 12 was injected into the chamber of the working electrode substrate. The working electrode substrate was allowed to stand for 1.5 hours at room temperature, and the biotinylated DNA 97 was bonded to the streptavidin 93 immobilized on the working electrode (refer to FIG. 12). Thereafter, the working electrode was washed with 20 µL of TBS-T and 20 µL sterile purified water to remove unbonded labeled substance. The chamber was then removed, the working electrode was washed three times with 0.5 mL of sterile purified water, and dried.

### (3) Measuring the Photocurrent

Next, silicone rubber was arranged as a side wall 0.2 mm thick around the working electrode substrate. Then, 12 µL of the electrolyte obtained in fabrication example 13 was loaded into the cavity circumscribed by the silicone rubber and the working electrode substrate. The counter electrode substrate obtained in fabrication example 14 was placed onto the working electrode substrate to seal the cavity loaded with the electrolyte. Thus, the electrolyte was in contact with the working electrode, the counter electrode, and the reference electrode. The electrode leads were then connected to the ammeter.

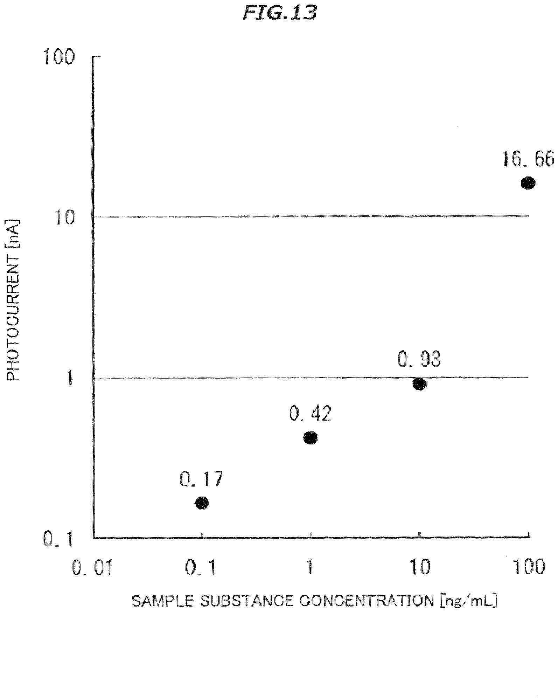

A voltage of 0 V was applied to the working electrode with the reference electrode as standard. At the same time, laser light (wavelength: 785nm, power 13mW) from a light source (Coherent Company, product name: Cube 785) was illuminated to the working electrode from the working electrode substrate side toward the counter electrode substrate. At this time, the laser light flashed (ON/OFF) with a predetermined period (1 Hz). Increament of the current flowing between the working electrode and the counter electrode was measured as a photocurrent when the laser light was emitted (turned ON) over 20 flashes of the laser light. FIG. 13 shows the results of the relationship between the photocurrent and the concentration of sample substance in experiment 3.

It can be understood from the results shown in FIG. 13 that noise is suppressed to such a degree that it does not obstruct detection of the photocurrent and the photocurrent increases dependently on the concentration of the sample substance when using the working electrode substrate provided with the adhesive layer 63, which is constituted by a silane coupling agent compound that binds to both the semiconductor layer 62 and the metal layer 64, interposed between the semiconductor layer 62 and the metal layer 64 when detecting an antigen as a sample substance via an antigen-antibody reaction using an antibody (Fab anti-mouse immunoglobulin antibody) as the capture substance.

### (Fabrication Example 15)

L-cysteine was added to TBS buffer solution so a concentration of 1 mM to obtain a solution C.

### (Fabrication Example 16)

Poly-L-lysine hydrochloride (Sigma Aldrich, catalog No. P2658-25 mg; α-poly-L-lysine hydrochloride, viscosity average molecular weight: 15000-30000 Da) was added to PBS to a concentration of 100 µM to obtain a solution D.

Examples of DNA detection using amino acids and polyamino acids as linker molecules are shown in example 8 and example 9 below.

### (Example 8)

### (1) Forming the Working Electrode Body

A substrate A1 was obtained by forming a semiconductor layer (working electrode body) constituted by a thin film (approximately 200 nm thickness) of tin-doped indium oxide on a substrate body of silicon dioxide (SiO₂), and forming a working electrode lead thereon via a spattering method for connecting the working electrode body with the ammeter. This thin film performed the functions of both a conductive layer and an electron acceptor layer.

### (2) Forming the Adhesive Layer

Silicone rubber (thickness 0.2 mm) was arranged as a partition to circumscribe the semiconductor layer of the substrate A1 obtained in step 1. Thereafter, 30 µL of solution C obtained in fabrication example 15 was titrated into the cavity circumscribed by the silicone rubber and the substrate A1, and the substrate A1 was allowed to rest overnight at 4°C. The substrate A1 was subsequently washed with TBS buffer solution and air dried. An adhesive layer constituted by a thin film of L-cysteine was thus formed on the semiconductor layer.

### (3) Forming the Metal Layer

A metal layer constituted by a thin gold film (approximately 2 nm thickness) was formed on the adhesive layer formed in step (2) via vacuum vapor deposition to obtain a substrate A2.

### (4) Immobilizing the Capture Substance

12 µL of the aqueous DNA solution obtained in fabrication example 3 was titrated on the metal layer of the substrate A2 obtained in step (3). Silicone rubber (0.1 mm thickness) was then disposed as a wall around the perimeter of the metal layer of the substrate A2. Thereafter, a cover glass was set on the silicone rubber to seal the cavity circumscribed by the substrate A2 and the silicone rubber. The substrate A2 was then allowed to stand overnight at 4°C to covalently bond the gold constituting the thin gold film and the thiolated DNA constituting the capture substance. The metal layer of the substrate A2 was thereafter washed with TBS buffer solution.
The surface of the substrate on which the DNA probe is immobilized is thereafter washed as described below to remove the DNA probe nonspecifically adhered to the semiconductor layer and thin metal film. A hybridization chamber (20 µL volume) was then placed on the silicon rubber. 20 µL of hybridization solution (PerfectHyb (trademark) Hybridization solution, manufactured by Toyobo Company, Ltd.) was injected into the cavity formed by the hybridization chamber. The substrate A2 was then allowed to stand for 2 hours in a thermoregulated bath (45°C). The wash liquid was subsequently discharged from the cavity. Then, 100 µL of hybridization solution was injected into the cavity, and the same operation was repeated. In this way a working electrode substrate was obtained.

### (Example 9)

A working electrode substrate was produced using identical operations to those of Example 8, with the exception that the solution D of fabrication example 16 was used rather than the solution C obtained in fabrication example 15 in Example 8.

### (Comparative Example 4)

A working electrode substrate was produced using identical operations to those of Example 8, with the exception that TBS buffer solution was used rather than the solution C obtained in fabrication example 15 in Example 8.

### (Comparative Example 5)

A working electrode substrate was produced using identical operations to those of Example 8, with the exception that no solution was used rather than the solution C obtained in fabrication example 15 in Example 8.

### (Experiment 4)

### (1) Hybridization Process

100 µL of hybridization aqueous solution or hybridization solution containing 10 nM of DNA labeled with AlexaFluor750 was injected into the hybridization chamber containing the mounted working electrode substrates obtained in examples 8 and 9, and comparative examples 4 and 5.
The working electrode substrate was then allowed to stand 4 hours in a thermoregulated bath (45°C). Thereafter, the hybridization chamber was subsequently removed upward from the electrode. The working electrode substrate was washed with washing solution (2xSSC liquid containing 0.01 wt% SDS), and washed with sterile purified water, and subsequently dried.

### (2) Metal Layer Stability Evaluation

Stability was visually evaluated for the metal layer on the working electrode of the working electrode substrates obtained in examples 8 and 9 and comparative examples 4 and 5 in the same manner as in experiment 1. Evaluation results are shown in Table 2.

### [Evaluation Criteria]

○ indicates the metal layer remains completely.
× indicates peeling of the metal layer is observed.

**Table 2**

| | Linker molecule | Evaluation |
|---|---|---|
| Example 8 | L - Cysteine | ○ |
| Example 9 | Poly-L-Lysine | ○ |
| Comparative Example 4 | T B S | × |
| Comparative Example 5 | - | × |

It can be understood from the results shown in Table 2, when an adhesive layer constituted by L-cysteine or poly-L-cysteine as a linker molecule was formed between the semiconductor layer and the metal layer (Examples 8 and 9), the metal layer showed no separation, and the metal layer was maintained in good condition. Thus, these results suggest that a working electrode substrate that uses an amino acid such as L-cysteine L or a polyamino acid such as poly-L-lysine, which are compounds that bind to both the semiconductor layer and the metal layer as a linker molecule, is capable of detecting sample substances with high degrees of detection sensitivity and reproducibility.

### (Experiment 5)

### (1) Hybridization Process

100 µL of hybridization aqueous solution or hybridization solution containing 10 nM of DNA labeled with AlexaFluor750 was injected into the hybridization chamber containing the mounted working electrode substrates obtained in examples 8 and 9, and comparative examples 4 and 5.
The working electrode substrate was then allowed to stand 4 hours in a thermoregulated bath (45°C). Thereafter, the hybridization chamber was removed upward from the electrode, The working electrode substrate was washed with washing solution (2xSSC liquid containing 0.01 wt% SDS), washed with sterile purified water, and subsequently dried.

### 2) Attracting the Sample Substance

After performing operation (1), silicon rubber was arranged as a side wall 0.2 mm thick around the working electrode substrate. Then, 12.5 µL of the dissolved attractant electrolyte obtained in fabrication example 13 was loaded into the cavity circumscribed by the silicone rubber and the working electrode substrate. The counter electrode substrate obtained in fabrication example 14 was placed onto the working electrode substrate to seal the cavity loaded with the dissolved attractant electrolyte. Thus, the dissolved attractant electrolyte was in contact with the gold thin film of the working electrode, the counter electrode, and the reference electrode. After resting in this state for 5 minutes at room temperature, the electrode leads were connected to the ammeter and the photocurrent was measured.

### (2) Measuring the Photocurrent

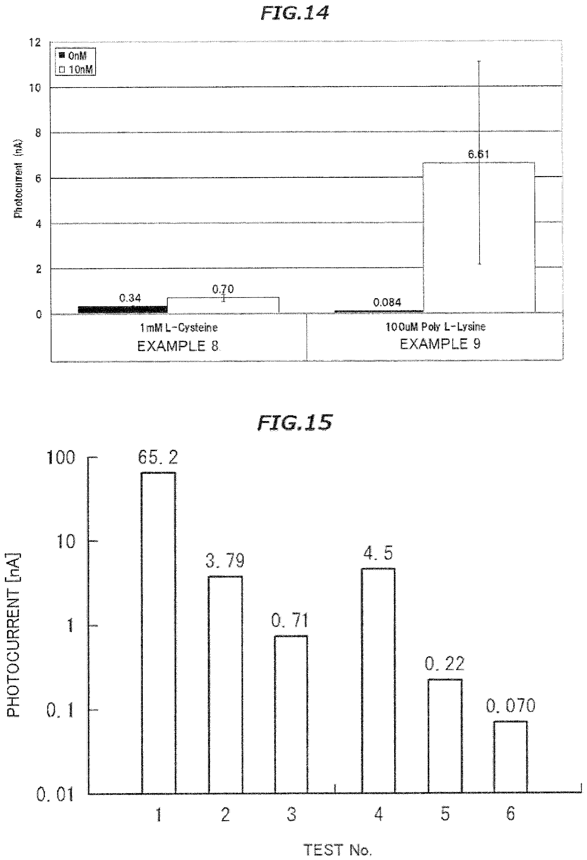

A voltage of 0 V was applied to the working electrode with the reference electrode as standard. At the same time, laser light (wavelength: 785 nm, output 13 mW) emitted from a light source was irradiated to the working electrode from the working electrode substrate side toward the counter electrode substrate. At this time, the laser light flashed (ON/OFF) with a predetermined period (1 Hz). The increment of current flowing between the working electrode and the counter electrode was measured as photocurrent when the laser light was emitted (turned ON) over 20 flashes of the laser light. In Experiment 5, the working electrode substrates obtained in Examples 8 and 9 were used, and the results of measuring the photocurrent are shown in FIG. 14. In the figure, the black bars indicate the photocurrent when the hybridization aqueous solution (concentration of AlexaFluor750 labeled-DNA 0nM) was used, and the white bars indicate the photocurrent when hybridization aqueous solution containing 10 nM of DNA labeled with AlexaFluor750 was used. In the figures, error bars indicate the standard deviation of the photocurrent obtained in three points on the working electrode. Moreover, in the figure, "DNA" indicates the labeled DNA AlexaFluor750.
It can be understood from the results shown in Figure 14 that the photocurrent derived from the AlexaFluor750 which is labeled in AlexaFluor750-labeled DNA can be well detected when using either the working electrode substrate having poly-L-lysine as a linker molecule (example 9), or a working electrode substrate having L-cysteine as a linker molecule (example 8). Thus, these results suggest sample substances can be well detected using a working electrode substrate having an amino acid such as L-cysteine and the like as a linker molecule, or a polyamino acid such as poly-L-lysine as a linker molecule. It can also be understood from the results shown in Figure 14 that the photocurrent based on AlexFluor750 using the working electrode substrate having poly-L-lysine as a linker molecule (example 9) is greater than the photocurrent based on AlexaFluor750 using the working electrode substrate having L-cysteine as a linker molecule (example 8). Moreover, the signal-to-noise ratio (S/N) when using the working electrode substrate having poly-L-lysine as a linker molecule (example 9) was greater than the S/N when using the working electrode substrate having L-cysteine as a linker molecule. Thus, from the viewpoint of high sensitivity in detecting a sample substance, the linker molecule is preferably poly-L-lysine rather than L-cysteine.

### (Test Example 1)

### (1) Fabricating the Working electrode Substrate

A working electrode 1 was obtained by forming a thin film (approximately 200 nm thickness) indium tin oxide (ITO), and a thin film (approximately 100 nm thickness) of antimony-doped tin oxide (ATO) on the surface of a substrate body of silicon dioxide (SiO₂) via a spattering method. A thin film (thickness approximately 10 nm) of titanium oxide (TiO₂) was formed on the working electrode 1 to obtain a working electrode 2. The titanium oxide was used to attach the metal strongly to the semiconductor as in conventional use.

### (2) Measuring the Photocurrent

Next, silicon rubber was arranged as a side wall 0.2 mm thick around the working electrode substrate. Then, 12 µL of the electrolyte obtained in fabrication example 13 was loaded into the cavity circumscribed by the silicon rubber and the working electrode substrate. The counter electrode substrate obtained in fabrication example 14 was placed from above onto the working electrode substrate to seal the cavity loaded with the electrolyte. Thus, the electrolyte was in contact with the working electrode, the counter electrode, and the reference electrode. The electrode leads were then connected to the ammeter.

A voltage of 0 V was applied to the working electrode with the reference electrode as standard. At the same time, laser light of 473 nm wavelength emitted from a light source (output 13mW, Photop Suwtech Company, product name DPBL-9050), laser light of 640 nm wavelength (output 13 mW, Coherent Company, product name Cube 640), or laser light of 785 nm wavelength (output 13 mW, Coherent Company, product name Cube 785) was irradiated to the working electrode from the working electrode substrate side toward the counter electrode substrate. At this time, the laser light flashed (ON/OFF) with a predetermined period (1 Hz). The increment of current flowing between the working electrode and the counter electrode was measured as a photocurrent when the laser light was emitted (turned ON) over 20 flashes of the laser light. FIG. 15 shows the results of measured photocurrent in Test Example 1. Test No. 1 shows the photocurrent derived from the working electrode2 with 473nm wavelength laser light illumination; test no. 2 shows the photocurrent derived from the working electrode2 with 640nm wavelength laser light illumination; test no. 3 shows the photocurrent derived from the working electrode2 with 785nm wavelength laser light illumination; test no. 4 shows the photocurrent derived from the working electrode1 with 473nm wavelength laser light illumination; test no. 5 shows the photocurrent derived from the working electrode1 with 640nm wavelength laser light illumination; and test no. 6 shows the photocurrent derived from the working electrode1 with 785nm wavelength laser light illumination.

It can be understood from the results shown in FIG. 15 that the value of the photocurrent derived from the working electrode increases more than 10 times in the working electrode 2 (test nos. 1 through 3) which has a titanium oxide thin film compared to the working electrode 1 (test nos. 4 through 6) which does not have a titanium oxide thin film. These results suggest that the photocurrent derived from the working electrode increases and the S/N is thereby markedly reduced when using a titanium oxide thin film as an adhesive layer to attach the metal layer to the semiconductor layer in the working electrode substrate used in the detection of a photocurrent. This further suggests that titanium, which is conventionally used to attach a semiconductor body to a metal, may be difficult to use as an adhesive layer to maintain the stability of the metal layer in a working electrode substrate used to detect a photocurrent.

It can be understood from these results that the electrode of the present invention provides stronger adhesion between the semiconductor layer and the metal layer via an adhesive layer by providing an adhesive layer constituted by linker molecules between the semiconductor layer and the metal layer. As a result, the metal layer is unlikely to peel away when the capture substance captures the test substance during detection of the test substance, and when a blocking process is performed to improve detection sensitivity. It can be understood that a sample substance can be detected with a high degree of reproducibility using the electrodes of the present invention in photochemical detection methods.

## Claims

1. A photocurrent detection electrode used to photoelectrochemically detect a test substance that releases electrons by photoexcitation, comprising:
a semiconductor body comprising a semiconductor which receives the electrons originating from the test substance irradiated by excitation light;
an adhesive layer containing linker molecules formed on the electrode body; and
a metal layer comprising a metal formed on the adhesive layer.

2. The electrode according to claim 1, wherein
a capture substance for capturing the test substance is immobilized on the metal layer.

3. The electrode according to claim 2, wherein
a part of the metal layer, where the capture substance is not immobilized, is blocked by a blocking agent.

4. The electrode according to any one of claim 1 to 3, wherein
the linker molecule comprises at least one selected from groups including; a silane coupling agent;
a titanium coupling agent; and
compounds represented by Equation (1):
R¹-X-R² (1)
(where R1 represents a silanol group, a phosphoric acid group, carboxyl group or a thiol group, R2 represents an amino group, thiol group, alkyl group having 1 to 4 carbon atoms, hydroxyl, carboxyl, sulfonic acid group, an epoxy group, methacryloyl group; acryloyl group or vinyl group; X represents equation (2):
(CH₂)ₘ (2)
(where m represents an integer 1 to 20) or equation (3): 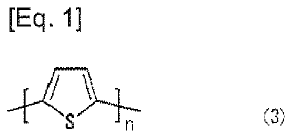 (where n represents an integer 1 to 100), and
there is an amino bond, ester bond, ether bond, or dithiol bond represented by equation (1)).

5. The electrode according to any one of claim 1 to 3, wherein
the linker molecule is an amino acid or a compound comprising an amino acid residue.

6. The electrode according to any one of claim 1 to 5, wherein
the metal layer comprises a metal that is dissolved by the electrolyte used in the photoelectrochemical detection of the test substance from which the electrons originate by photoexcitation.

7. The electrode according to claim 6, wherein
the metal layer is at least one metal selected from groups including gold and palladium.

8. A working electrode substrate used to photoelectrochemically detect a test substance that releases electrons by photoexcitation, comprising:
a substrate body; and
a photocurrent detection electrode; wherein
the photocurrent detection electrode comprises:
a semiconductor body, formed on the substrate body, and comprising a semiconductor which receives the electrons originating from the test substance irradiated by excitation light;
an adhesive layer containing linker molecules formed on the electrode body; and
a metal layer comprising a metal formed on the adhesive layer.

9. The working electrode substrate according to claim 8, wherein
a capture substance for capturing the test substance is immobilized on the metal layer.

10. The working electrode substrate according to claim 9, wherein
a part of the metal layer, where the capture substance is not immobilized, is blocked by a blocking agent.

11. A method of manufacturing a photocurrent detection electrode used to photoelectrochemically detect a test substance that releases electrons by photoexcitation, comprising:
a step of forming an adhesive layer containing linker molecules on an electrode body configured by a semiconductor which accepts the electrons released via photoexcitation, and
a step of forming as metal layer on the adhesive layer.

12. The method according to claim 11, further comprising:
a step of immobilizing a capture substance for capturing the test substance on the metal layer.

13. The method according to claim 12, further comprising:
a step of blocking a part of the metal layer, where the capture substance is not immobilized, by a blocking agent.
